# EUROPEAN PATENT SPECIFICATION

(11) **EP 2 205 259 B1**
(45) Date of publication and mention of the grant of the patent: **10.12.2014**
(21) Application number: 08800421.3
(22) Date of filing: 02.10.2008
(51) Int. Cl.: A61K 38/02, A61P 29/00, A61P 37/02, A61P 37/06

(54) **MPM (MALLEABLE PROTEIN MATRIX) FOR USE IN THE TREATMENT OF ARTHRITIS, OBESITY, INSULIN RESISTANCE, TYPE 2 DIABETES, AUTOIMMUNE DISEASE, HYPERTENSION, AND HYPERLIPIDEMIA**
MPM (MALLEABLE PROTEIN MATRIX) ZUR VERWENDUNG ZUR BEHANDLUNG VON FETTSUCHT, INSULINRESISTENZ, DIABETES TYP 2, AUTOIMMUNKRANKHEITEN, HYPERTENSION UND HYPERLIPIDEMIE
MPM (MALLEABLE PROTEIN MATRIX) POUR SON UTILISITATION DANS LE TRAITEMENT DE L'ARTHRITE, L'OBESITE, LA RÉSISTANCE À L'INSULINE, LE DIABETE DE TYPE 2, LES MALADIES AUTOIMMUNES, L'HYPERTENSION ET L'HYPERLIPIDEMIE

(30) Priority: 02.10.2007 US 976983 P
(43) Date of publication of application: 14.07.2010
(73) Proprietor: Institut National de la Recherche Scientifique, Québec G1K 9A9 (CA)
(72) Inventor: BEAULIEU, Josée, Laval, Québec H7R 6G7 (CA); LAPOINTE, Jean-François, Montréal, Québec H2E 2C6 (CA); DUPONT, Claude, Blainville, Québec J7B 1L6 (CA); LEMIEUX, Pierre, Ste-Thérèse, Québec J7E 4Z2 (CA); SIMARD, Eric, Boisbriand, Québec J7H 2K9 (CA); TROTTIER, Eric, Mirabel, Québec J7J 1C9 (CA)
(74) Representative: Brown, David Leslie
(86) International application number: PCT/CA2008/001755
(87) International publication number: WO 2009/043171

(56) References cited:
- WO-A1-2006/084381
- WO-A2-03/053158
- BEAULIEU, J. ET AL.: 'Anti-Inflammatory Potential of a Malleable Matrix Composed of Fermented Whey Proteins and Lactic acid Bacteria in an Atopic Dermatitis Model.' JOURNAL OF INFLAMMATION. vol. 4, 21 March 2007, pages 1 - 10, XP008123437
- BEAULIEU, J. ET AL.: 'Immunomodulation by a Malleable Matrix Composed of Fermented Whey Proteins and Lactic Acid Bacteria.' vol. 10, no. 1, 01 March 2007, pages 67 - 72, XP008123583

## Description

### TECHNICAL FIELD

The invention relates to a malleable protein matrix (MPM) for use in the treatment of conditions wherein the MPM modulates the biological activity of IL-17-producing cells.

### BACKGROUND OF THE INVENTION

The immune system functions to protect individuals from infective agents, e.g., bacteria, multi-cellular organisms, and viruses, as well as from cancers. This system includes several types of lymphoid and myeloid cells such as monocytes, macrophages, dendritic cells (DCs), eosinophils, T cells, B cells, and neutrophils. These lymphoid and myeloid cells often produce signaling proteins known as cytokines. The immune response includes inflammation, i.e., the accumulation of immune cells systemically or in a particular location of the body. In response to an infective agent or foreign substance, immune cells secrete cytokines which, in turn, modulate immune cell proliferation, development, differentiation or migration. Immune response can produce pathological consequences, e.g., when it involves excessive inflammation, as in the autoimmune disorders.

The inflammatory processes are also implicated in various metabolic pathways, disease predispositions, ailments like allergy or disease initiation. The most important metabolic impact of inflammatory processes is probably the metabolic cascade associated with overweight and obesity. This cascade is responsible for the development of type 2 diabetes, hypertension or hyperlipidemia. Thus, a high level of inflammation or inflammatory cytokines, can promote the development of obesity, allergies, cancer, autoimmune diseases, non-alcoholic fatty liver disease (NAFLD), non-alcoholic steatohepatitis (NASH) with or without fibrosis, cirrhosis, hepatocellular carcinoma, Cushing's syndrome, type 2 diabetes, hypertension or hyperlipidemia. Then, the diminution of inflammatory processes can prevent the development of certain diseases or metabolic problems.

Naïve T cells respond to antigenic stimulation. In presence of interferon gamma (INF*γ*) and interleukin 12 (IL-12), CD4⁺ cells will differentiate into T helper type 1 cells (Th₁) for the promotion of cell-mediated immune response to intracellular pathogens. If interleukin 4 (IL-4) is present, then CD4⁺ cells will develop into T helper type 2 cells (Th₂) by producing IL-4, IL-5 and IL-13 for a humoral immune response to extra cellular pathogens. The combination of transforming growth factor (TGF-*β*1), interleukin 6 (IL-6) and interleukin 23 will make CD4⁺ cells develop into Th₁₇ cells based on their production of interleukin 17 (IL-17) which is not produce by Th₁ and Th₂ CD4⁺ cells. Their differentiation is also inhibited by INF*γ*, IL-4 and IL-35.

IL-17 is a proinflammatory cytokine that will enhances T cell priming and stimulates different cell types (fibroblasts, endothelial cells, macrophages and epithelial cells) to produce IL-1, IL-6, tumor necrosis factor (TNF-*α*), granulocyte-macrophage colony-stimulating factor (GM-CSF), nitric oxide synthase (NOS-2), metalloproteases and chemokines, leading to the induction of inflammation. Its expression is increased in patients with allergic and autoimmune diseases like rheumatoid arthritis, multiple sclerosis, inflammatory bowel disease (IBD) and asthma. Therefore, IL-17-producing cells (Th₁₇ cells) play an important role in the development of autoimmunity and allergic reaction (Iwakura et al., 2006, J. Clin. Inves. 116: 1218-1222).

Interleukin-23 (IL-23) promotes the expansion and survival of Th₁₇ cells that produce IL-17, IL-6 and TNF-*α*, but not INF-*γ* and IL-4. IL-23 is a heterodimeric cytokine comprised of two subunits, p19 which is unique to IL-23, and p40, which is shared with IL-12. The p19 subunit is structurally related to IL-6, granulocyte-colony stimulating factor (G-CSF) and the p35 subunit of IL-12. IL-23 mediates signaling by binding to a heterodimeric receptor, comprising IL-23R and IL-12*β*1, which is shared by the IL-12 receptor. A number of early studies demonstrated that the consequences of a genetic deficiency in p40 (p40 knockout mouse; p40KO mouse) were more severe than those found in a p35KO mouse. Some of these results were eventually explained by the discovery of IL-23, and the finding that the p40KO prevents not only expression of IL-12, but also of IL-23.

Recent studies, through the use of p40KO mice, have shown that blockade of both IL-23 and IL-12 is an effective treatment for various inflammatory and autoimmune disorders. However, the blockade of IL-12 through p40 appears to have various systemic consequences such as increased susceptibility to opportunistic microbial infections.

In the context of Th₁₇, blocking IL-23 or its downstream factor (IL-17, IL-6), but not the Th₁ pathway (IL-12, INF*γ*) may be a novel therapeutic target for the treatment of chronic inflammatory diseases as seen in animals models (Iwakura et al., 2006, J. Clin. Inves. 116: 1218-1222).

Interleukin 27 (IL-27) is produced by activated antigen-presenting cells and has been shown to negatively regulate the development of Th₁₇ cells during chronic inflammation of the central nervous system.

Interleukin 21 (IL-21) is selectively produced by Th₁₇ cells. IL-21 induces expression of retinoid-related orphan receptor gamma (RORγt), IL-17A, and IL-17F leading to an autocrine regulation of IL-17 production which serves to promote and sustain Th₁₇ cells differentiation (Wei et al. 2007, J Biol. Chem., Sep 20).

Interleukin 22 (IL-22) is also produced by Th₁₇ cells and acts cooperatively with IL-17A or IL-17F to enhance expression of antimicrobial peptides associated with host defense and acts as an effector cytokine of the Th₁₇ cells lineage (Liang et al. 2006, J Exper. Med. 203: 2271-2279).

Prostaglandins (PGs), particularly PGE₂, which is found at high concentrations in inflammatory foci, has been implicated as a proinflammatory agent. Cyclooxygenase 2 (COX-2) and microsomal PGE synthase 1 (mPGES-1), both of which are enzymes involved in PGE₂ generation, are highly expressed in the synovium of patients with rheumatoid arthritis (RA) and other inflammatory diseases. It has been reported that PGE₂ enhances dendritic cells-derived IL-6 production and induces a shift in the IL-23/IL-12 balance in favor of IL-23, resulting in increased IL-17 production through the amplification of Th₁₇ cells. High levels of PGE₂ also exacerbate the inflammatory process in inflammatory bowel disease through the IL-23 /IL-17 axis.

Whey-derived products are known for their positive effects including modulation of the immune system (Beaulieu et al., 2006, Therapy 3: 1-10). It has been demonstrated that these products exhibit an antioxidant potential by increasing glutathione contents, act as anti-inflammatory or anti-allergic agents and exhibit immunomodulatory potential.

Moreover, many dairy minerals such as calcium possess immunomodulatory properties. In addition to effect on hypertension, hypercholesterolemia and obesity, calcium increases activities of immune cells. Calcium increases the expression of IL-2 receptor on T cells as well as IL-2 production following stimulation of these cells. Other dairy minerals and vitamins like magnesium, zinc, iron, phosphorus, manganese, copper, selenium, niacin, riboflavin or vitamin C, already recognized for their metabolic roles, can also have an impact in synergy or alone, on the immunomodulatory properties of a dairy based product.

Microorganisms are present in many foods and are frequently used as probiotics to improve some biological functions in the host. Clinical trials have demonstrated that selected probiotic strains can influence the composition of the intestinal microflora and modulate the host immune system. Pre-, pro- and synbiotics offer both protection against and cure a variety of endemic and acute diseases. More particularly, the lactic acid bacteria (LAB) are known for their several beneficial effects on health.

The genus *Lactobacillus,* the most studied of these probiotics, is commonly used in the milk fermentation process. It has also been demonstrated that the effects of probiotics in synergy with food ingredients could be more intense than the probiotics alone. The effects of LAB are strain-dependent, but many *Lactobacillus* strains act on Peyer's patches to stimulate the production of secretory IgA, help phagocytosis and exhibit anti-inflammatory action by regulation of cytokine production and anti-allergic potential by reduction of IgE production. LAB increase the production of a large variety of cytokines depending on the strain used; some increase the Th₁ profile, while others increase the Th₂ profile, and represent a potential therapeutic agent for the treatment of chronic inflammatory diseases.

Lactic acid bacteria possess many constituents, such as DNA and exopolysaccharides (EPS), which exhibits many effects on immunity. LAB DNA increases production and activities of NK cells and lymphocytes, increases cytokine production and stimulates intestinal immunity by positive effect on Peyer patches. Some LAB strains produce EPS that exhibits immune effects such as immunostimulatory and anti-inflammatory properties. EPS increase cytokine production and stimulate phagocytosis by macrophages indicating a stimulation of innate immunity. EPS also stimulate B cells to produce specific antibodies production.

It would thus be highly desirable to be provided with a whey-derived product for the treatment of chronic inflammatory diseases. More specifically, it would be highly desirable to be provided with a whey-derived product that modulates the biological activity of IL-17-producing cells (Th₁₇ cells).

### SUMMARY OF THE INVENTION

The present invention is defined in and by the appended claims.

The malleable protein matrix (MPM) described herein immunomodulates an immune response in a subject.

The present disclosure describes an immunosuppressing composition or anti-inflammatory composition comprising an effective amount of MPM and a pharmaceutically acceptable carrier, wherein said composition modulates the biological activity of IL-17-producing cells.

The present disclosure also describes a method for preventing, alleviating or treating the condition of a patient by modulating the biological activity of IL-17-producing cells, said method comprising administering to said subject an effective amount: of a malleable protein matrix, and wherein said condition is selected form the group consisting of inflammation, infection, hypertension, arthritis, psoriasis, inflammatory bowel disease, multiple sclerosis, systemic lupus erythematosus, type 1 diabetes, obesity, insulin resistance, type 2 diabetes, cancer, obesity, allergies, autoimmune disease, hypertension, non-alcoholic fatty liver disease (NAFLD), non-alcoholic steatohepatitis (NASH) with or without fibrosis, cirrhosis, hepatocellular carcinoma, Cushing's syndrome and hyperlipidemia.

The present disclosure also describes the use of MPM for preventing, alleviating or treating the condition of a patient by modulating the biological activity of IL-17-producing cells, said method comprising administering to said subject an effective amount of a malleable protein matrix, and wherein said condition is selected form the group consisting of inflammation, infection, hypertension, arthritis, psoriasis, inflammatory bowel disease, multiple sclerosis, systemic lupus erythematosus, type 1 diabetes, obesity, insulin resistance, type 2 diabetes, cancer, obesity, allergies, autoimmune disease, hypertension, nonalcoholic fatty liver disease (NAFLD), non-alcoholic steatohepatitis (NASH) with or without fibrosis, cirrhosis, hepatocellular carcinoma, Cushing's syndrome and hyperlipidemia.

The present disclosure also describes the use of MPM in the manufacture of a medicament for preventing, alleviating or treating the condition of a patient by modulating the biological activity of IL-17-producing cells, said method comprising administering to said subject an effective amount of a malleable protein matrix, and wherein said condition is selected form the group consisting of inflammation, infection, hypertension, arthritis, psoriasis, inflammatory bowel disease, multiple sclerosis, systemic lupus erythematosus, type 1 diabetes, obesity, insulin resistance, type 2 diabetes, cancer, obesity, allergies, autoimmune disease, hypertension, non-alcoholic fatty liver disease (NAFLD), non-alcoholic steatohepatitis (NASH) with or without fibrosis, cirrhosis, hepatocellular carcinoma, Cushing's syndrome and hyperlipidemia.

MPM modulates the biological activity of IL-17-producing cells.

In an embodiment, the subject is a human or an animal.

The immune response can be selected from the group consisting of an inflammatory response, an autoimmune response, a metaflammation, an infection and a wound healing. Specifically, the infection is selected from the group consisting of a bacterial infection, a viral infection and fungal infection.

The subject can have a disorder selected from the group consisting of arthritis, psoriasis, inflammatory bowel disease, multiple sclerosis, systemic lupus erythematosus, type 1 diabetes, obesity, insulin resistance, non-alcoholic fatty liver disease (NAFLD), non-alcoholic steatohepatitis (NASH) with or without fibrosis, cirrhosis, hepatocellular carcinoma, Cushing's syndrome and type 2 diabetes.

The subject may have cancer.

The subject may have allergies.

The MPM can reduce expression of a cytokine selected from the group consisting of IL-12, IL-1*β*, IL-6, transforming growth factor (TNF*α*), granulocyte-macrophage colony-stimulating factor (GM-CSF) and interferon gamma (IFN*γ*). Alternatively, the MPM can increase the expression of a cytokine selected from the group consisting of IL-2, IL-23, TNF*α*, GM-CSF and IL-18.

The MPM can decrease levels of plasma triglycerides or cholesterol.

The MPM can control, help control, ameliorate, or improve weight or body composition.

The MPM can decrease the expression of cyclooxygenase 2 (COX-2) or production of prostaglandin E2 (PGE₂).

The MPM can prevent hypertension.

The MPM can increase insulin sensitivity or glucose homeostasis.

The MPM can increase polymorphonuclear cells or CD4+ cells population.

The composition can further comprise an immunosuppressive drug or an anti-inflammatory drug.

The composition can be used as a medicament, dietary supplement, functional food, cosmeceutical supplement or medical food.

### BRIEF DESCRIPTION OF THE DRAWINGS

Figs. 1A and 1B illustrate the effect of malleable protein matrix (MPM) on stimulation of the immune pathways in healthy animals;
Fig. 2A illustrates the effect of MPM on systemic cytokine profile in an Atopic Contact Dermatitis (ACD) mice model;
Fig. 2B illustrates the effect of MPM on the relative expression of 20 Th-17 pathway-related genes in oxazolone-treated ears in an Atopic Contact Dermatitis (ACD) mice model;
Figs. 3A and 3B illustrate the effect of MPM on local cytokine profile in a rheumatoid arthritis-like model (air pouch model);
Fig. 4 illustrates the effect of MPM on IL-23 production in a mild inflammatory animal model;
Fig. 5A illustrates the effect of MPM on body weight gain of mice on a high carbohydrate diet;
Fig. 5B illustrates the effect of MPM on epididymal fat pads weight of mice on a high carbohydrate diet;
Fig. 5C illustrates the effect of MPM on body composition;
Fig. 6A illustrates the effect of MPM on total plasma triglycerides level in a poloxamer-induced hyperlipidemia rat model;
Fig. 6B illustrates the effect of MPM on total plasma cholesterol level in a poloxamer-induced hyperlipidemia rat model;
Fig. 6C illustrates the effect of MPM on the reduction percentage of triglycerides and cholesterol level in total plasma in a poloxamer-induced hyperlipidemia rat model;
Fig. 7A illustrates the effect of MPM on the fasting blood glucose tolerance test (OGTT) of rats on a high fructose diet;
Fig. 7B illustrates the effect of MPM on the plasma glucose area under the curve (AUC) of rats on a high fructose diet;
Fig. 7C illustrates the effect of an alternated treatment of MPM (30 days) or Water (30 days) on the plasma glucose area under the curve (AUC) of rats on a high fructose diet;
Fig. 8 illustrates the effect of MPM on systolic blood pressure (SBP) of spontaneously hypertensive rats (SHR);
Fig. 9A illustrates the effect *in vitro* of MPM on the expression of different genes on human keratinocytes (HEKA);
Fig. 9B illustrates the effect of MPM on PGE₂ production in a EPI-200 model (Mattek);
Fig. 10A illustrates the effect of MPM in an Atopic Contact Dermatitis (ACD) model for a preventive approach
Fig. 10B illustrates the effect of MPM in an Atopic Contact Dermatitis (ACD) model for a therapeutic approach; and
Fig. 11 illustrates the effect of MPM on stimulation of the body natural defenses in healthy animals.

### DETAILED DESCRIPTION OF THE PREFERRED EMBODIMENT

In the present disclosure, there is described a malleable protein matrix generated from the fermentation of whey by lactic acid bacteria that modulates the biological activity of IL-17-producing cells (Th₁₇ cells), particularly in inflammatory, autoimmune and proliferative disorders.

The present invention is based on the observation that modulation of the biological activity of Th-_{I7} cells can stimulate the immune system and natural defenses in healthy individual and act as an inhibitor of inflammation, autoimmunity, and abnormal proliferation. Then, modulation of the biological activity of Th-_{I7} cells may prevent the emergence, reduce the frequency or severity, promote the healing or prevent the development of different health problems.

A number of cytokines have a role in the pathology or repair of neurological disorders. IL-6, IL-17, INF-γ and GM-CSF have been associated with multiple sclerosis. IL-1α, IL-1β, and TGF-β1 play a role in amyotrophic lateral sclerosis (ALS), Parkinson's disease, and Alzheimer's disease. TNFα, IL-1β, IL-6, IL-8, INFγ, and IL-17 appear to modulate response to brain ischemia. Vascular endothelial cell growth factor (VEGF) is associated with ALS.

Inflammatory bowel disorders, e.g., Crohn's disease, ulcerative colitis, celiac disease, and irritable bowel syndrome are mediated by cells of the immune system and by cytokines. For example, Crohn's disease is associated with increased IL-12 and INF*γ*, while ulcerative colitis is associated with increased IL-5, IL-13, and transforming growth factor-beta (TGF*β*). IL-17 expression also increases in Crohn's disease and ulcerative colitis.

Inflammatory diseases of the skin, joints, CNS, as well as proliferative disorders elicit similar immune responses. Thus, the decrease in Th₁₇ cells provides inhibition of these immune mediated inflammatory disorders without compromising the host ability to fight systemic infections. Antagonizing Th₁₇ cells should relieve the inflammation associated with inflammatory bowel disease, Crohn's disease, Ulcerative Colitis, rheumatoid arthritis, psoriatic arthritis, psoriasis and atopic dermatitis. The use of Th₁₇ cells inhibitors will also provide inhibition of proliferative disorders, e.g., cancer and autoimmune disorders e.g., multiple sclerosis, type 1 diabetes, and systemic lupus erythematosus (SLE).

Metaflammation or "chronic" inflammation is characterized by abnormal cytokine production, increased acute-phase reactants and other mediators, and activation of a network of inflammatory signaling pathways. Experimental, epidemiological and clinical evidence links inflammation to the development of metabolic diseases and/or complications that emerge from these pathologies. The decrease in Th₁₇ cells provides inhibition of metaflammation particularly in the context of obesity, insulin resistance, type 2 diabetes, hypertension, fatty liver disease, atherosclerosis, degenerative disorders and airway disease. Some cancers are closely associated with metaflammation. This benefic modulation can be also useful to control, help control, improve or ameliorate weight and body composition.

Obviously, adipose tissue plays an important role in controlling whole-body homeostasis as the regulatory tissue modulating glucose and lipid homeostasis in humans. Moreover, chronic inflammation has a profound effect on adipocytes activity. In the lean state, small adipocytes efficiently store fatty acids as triglyceride (TG). In this condition, the insulin-stimulated glucose uptake is normal. Excess caloric intake leads to metabolic overload, increased TG input and adipocyte enlargement. When overloading with TG, hypertrophy of adipocytes and increased secretion of macrophage chemoattractants occurs resulting in additional macrophages in the adipose tissue. This recruitment in turn results in a pro-inflammatory state in obese adipose tissue. Infiltrating macrophages secrete large amounts of tumor necrosis factor-*α* (TNF-*α*), which results in a chronic inflammation state with impaired TG deposition and increased lipolysis. The excess of circulating TG and free fatty acids results in the accumulation of activated lipids in the muscle, disrupting functions such as insulin-stimulated glucose transport, leading to insulin resistance and type 2 diabetes, and ectopic storage of lipid within liver, and other non-adipose tissues.

Obesity can lead to a chronic inflammatory state within adipose tissue depots, which can cause adipocyte dysfunctions. Dysfunctions in adipose tissue metabolism have a direct impact on lipid and glucose homeostasis. Adipose dysfunctions in obesity include secretions of abnormal levels of cytokines linked to insulin resistance, impairments in triglyceride storage and increases in lipolysis. These abnormalities in turn can contribute to increased fatty acids in the circulation and lead to an overload of fatty acids in the skeletal muscle and the liver. Preventing the recruitment of immune cells in the adipose tissue would prevent obesity, insulin resistance, type 2 diabetes, fatty liver disease, metabolic syndrome and cardiovascular disease.

Plasma free fatty acid levels are elevated in obesity. Free fatty acid accumulation in liver produces low-grade inflammation through the activation of the nuclear factor-kappaB mediated by the TLR-4 pathway. This pathway is essential for the development of innate immunity to pathogens. The liver is sensing the excess of nutriments (free fatty acids) like infectious pathogens and uses the same signaling pathway (TLR-4) resulting in the release of several proinflammatory (TNF-a, IL-1b, IL-6) and proatherogenic cytokines (MCP-1). Thus, elevated free fatty acid levels (due to obesity or to high-fat feeding) cause insulin resistance in liver, which contributes to the development of type 2 diabetes, and produce low-grade inflammation, which contributes to the development of atherosclerotic vascular diseases, nonalcoholic fatty liver disease and metabolic syndrome. Modulating the free fatty acids-related release of proinflammatory cytokines in the liver would prevent those diseases.

Thus, that modulation of the biological activity of Th₁₇ cells can be used in a treatment approach for multiple purposes or in a preventive approach for the stimulation of natural defenses in healthy individual or as an inhibitor of inflammation. In a preventive approach, modulation of the biological activity of Th₁₇ cells may prevent the emergence, reduce the incidence or severity, promote the healing or prevent the development of different health problems. Like for cancer, allergy, obesity, Crohn's disease, fatty liver disease, psoriasis, multiple sclerosis, amyotrophic lateral sclerosis (ALS), Parkinson's disease, Alzheimer's disease, brain ischemia, ulcerative colitis, celiac disease, irritable bowel syndrome, psoriatic arthritis, atopic dermatitis, type 1 diabetes, systemic lupus erythematosus, insulin resistance, type 2 diabetes, hypertension, atherosclerosis, degenerative disorders, airway disease, non-alcoholic fatty liver disease (NAFLD), non-alcoholic steatohepatitis (NASH) with or without fibrosis, cirrhosis, hepatocellular carcinoma, Cushing's syndrome and the like. The reduction of inflammatory processes can reduce the risk of occurrence, the severity or promote the healing.

A whey-derived ingredient, the malleable protein matrix (MPM), is produced from fermented residual whey obtained from the cheese industry (see WO 03/053158, the entire content of which is hereby incorporated by reference). The MPM is obtained by triggering agglomeration of whey proteins, which are then retrieved by various means. Following the agglomeration, the resulting matrix is retrieved by filtration, centrifugation or with any other methods allowing such retrieval. The protein agglomeration can be triggered by, but not limited to, a modulation of pH, temperature, the addition of salts, the addition of proteolytic enzymes, the addition of flocculent or the combination of all or some of those methods.

The malleable protein matrix (MPM) is produced with the help of a fermentation process of whey using lactic acid bacteria (LAB). It has the appearance of a malleable gel cream-coloured and of neutral odour and taste. The product has been classified as a whey proteins hydrolysate by the Chemical Abstract Service (CAS) and given the number 308074-13-7.

The preferred microorganism used in the fermentation process of whey is a pure strain of lactobacillus isolated from a consortium obtained from Kefir grain, R2C2 (Accession Number 041202-3, National Microbiology Laboratory, Health Canada, 1015 Arlington Street, Winnipeg, Manitoba, Canada, R3E 3R2; WO 03/053158). The process can integrate a variety of other microorganisms either alone or in combination, selected from the group consisting of *Bifidobacterium adolescentis, Bifidobacterium angulatum, Bifidobacterium animalis, Bifidobacterium asteroides, Bifidobacterium bifidum, Bifidobacterium boum, Bifidobacterium breve, Bifidobacterium catenulatum, Bifidobacterium choerinum, Bifidobacterium coryneforme, Bifidobacterium cuniculi, Bifidobacterium dentium, Bifidobacterium gallicum, Bifidobacterium gallinarum, Bifidobacterium indicum, Bifidobacterium infantis, Bifidobacterium longum, Bifidobacterium longum* DJO10A, *Bifidobacterium longum* NCC2705, *Bifidobacterium magnum, Bifidobacterium merycicum, Bifidobacterium minimum, Bifidobacterium pseudocatenulatum, Bifidobacterium pseudolongum, Bifidobacterium pseudolongum subsp. globosum, Bifidobacterium pullorum, Bifidobacterium ruminantium, Bifidobacterium saeculare, Bifidobacterium scardovii, Bifidobacterium subtile, Bifidobacterium suis, Bifidobacterium thermacidophilum, Bifidobacterium thermacidophilum subsp. suis, Bifidobacterium thermophilum, Bifidobacterium urinalis, Lactobacillus acetotolerans, Lactobacillus acidipiscis, Lactobacillus acidophilus, Lactobacillus agilis, Lactobacillus algidus, Lactobacillus alimentarius, Lactobacillus amylolyticus, Lactobacillus amylophilus, Lactobacillus amylovorus, Lactobacillus animalis, Lactobacillus arizonensis, Lactobacillus aviarius, Lactobacillus bifermentans, Lactobacillus brevis, Lactobacillus buchneri, Lactobacillus casei, Lactobacillus cellobiosus, Lactobacillus coleohominis, Lactobacillus collinoides, Lactobacillus coryniformis, Lactobacillus coryniformis subsp. coryniformis, Lactobacillus coryniformis subsp. torquens, Lactobacillus crispatus, Lactobacillus curvatus, Lactobacillus cypricasei, Lactobacillus delbrueckii, Lactobacillus delbrueckii subsp. bulgaricus, Lactobacillus delbrueckii subsp. delbrueckii, Lactobacillus delbrueckii subsp. lactis, Lactobacillus durianis, Lactobacillus equi, Lactobacillus farciminis, Lactobacillus ferintoshensis, Lactobacillus fermentum, Lactobacillus fornicalis, Lactobacillus fructivorans, Lactobacillus frumenti, Lactobacillus fuchuensis, Lactobacillus gallinarum, Lactobacillus gasseri, Lactobacillus graminis, Lactobacillus hamsteri, Lactobacillus helveticus, Lactobacillus helveticus subsp. jugurti, Lactobacillus heterohiochii, Lactobacillus hilgardii, Lactobacillus homohiochii, Lactobacillus intestinalis, Lactobacillus japonicus, Lactobacillus jensenii, Lactobacillus johnsonii, Lactobacillus kefir, Lactobacillus kefiri, Lactobacillus kefiranofaciens, Lactobacillus kefirgranum, Lactobacillus kimchii, Lactobacillus kunkeei, Lactobacillus leichmannii, Lactobacillus letivazi, Lactobacillus lindneri, Lactobacillus malefermentans, Lactobacillus mali, Lactobacillus maltaromicus, Lactobacillus manihotivorans, Lactobacillus mindensis, Lactobacillus mucosae, Lactobacillus murinus, Lactobacillus nagelii, Lactobacillus oris, Lactobacillus panis, Lactobacillus pantheris, Lactobacillus parabuchneri, Lactobacillus paracasei, Lactobacillus paracasei subsp. paracasei, Lactobacillus paracasei subsp. tolerans, Lactobacillus parakefiri, Lactobacillus paralimentarius, Lactobacillus paraplantarum, Lactobacillus pentosus, Lactobacillus perolens, Lactobacillus plantarum, Lactobacillus pontis, Lactobacillus psittaci, Lactobacillus reuteri, Lactobacillus rhamnosus, Lactobacillus ruminis, Lactobacillus sakei, Lactobacillus sakei* L45, *Lactobacillus salivarius, Lactobacillus salivarius subsp. salicinius, Lactobacillus salivarius subsp. salivarius, Lactobacillus sanfranciscensis, Lactobacillus sharpeae, Lactobacillus* sp. NGRI 0001, *Lactobacillus suebicus, Lactobacillus thermotolerans, Lactobacillus vaccinostercus, Lactobacillus vaginalis, Lactobacillus vermiforme, Lactobacillus versmoldensis, Lactobacillus zeae, Lactococcus garvieae, Lactococcus lactis, Lactococcus lactis subsp. cremoris, Lactococcus lactis subsp. hordniae, Lactococcus lactis subsp. lactis, Lactococcus lactis subsp. lactis bv. diacetylactis, Lactococcus piscium, Lactococcus plantarum, Lactococcus raffinolactis, Leuconostoc argentinum, Leuconostoc carnosum, Leuconostoc citreum, Leuconostoc fallax, Leuconostoc ficulneum, Leuconostoc fructosum, Leuconostoc gasicomitatum, Leuconostoc gelidum, Leuconostoc inhae, Leuconostoc kimchii, Leuconostoc lactis, Leuconostoc mesenteroides, Leuconostoc mesenteroides subsp. cremoris, Leuconostoc mesenteroides subsp. dextranicum, Leuconostoc mesenteroides subsp. mesenteroides, Leuconostoc mesenteroides subsp. mesenteroides* ATCC 8293, *Leuconostoc pseudomesenteroides, Propionibacterium acidipropionici, Propionibacterium acnes, Propionibacterium australiense, Propionibacterium avidum, Propionibacterium cyclohexanicum, Propionibacterium freudenreichii, Propionibacterium freudenreichii subsp. freudenreichii, Propionibacterium freudenreichii subsp. shermanii, Propionibacterium granulosum, Propionibacterium jensenii, Propionibacterium lymphophilum, Propionibacterium microaerophilum, Propionibacterium propionicum, Propionibacterium thoenii, Saccharomyces delbrueckii, Saccharomyces cerevisiae, Saccharomyces unisporus, Saccharomyces globosus, Saccharomyces carlsbergensis, Kluyveromyces fragilis, Kluyveromyces bulgaricus, Kluyveromyces lactis, Torula holmii, Candida tenuis,* ES1 (Accession Number 041202-2, National Microbiology Laboratory, Health Canada, 1015 Arlington Street, Winnipeg, Manitoba, Canada, R3E 3R2), INIX (Accession Number 041202-4, National Microbiology Laboratory, Health Canada, 1015 Arlington Street, Winnipeg, Manitoba, Canada, R3E 3R2) and K2 (Accession Number 041202-1, National Microbiology Laboratory, Health Canada, 1015 Arlington Street, Winnipeg, Manitoba, Canada, R3E 3R2). The microorganisms are preferably homolactic but can be heterolactic.

The final product, MPM, is composed principally of fermented whey proteins and LAB. Also present in MPM are exopolysaccharides, dairy vitamins and minerals like niacin, riboflavin, calcium and a high proportion of peptides generated during the fermentation process. In healthy animals, a significant increase of blood polymorphonuclears (PMN) cells was observed following MPM administration indicating an immune response (Beaulieu et al., 2007, J Med Food, 10: 67-72). All components may explain partly the effect individually, but the synergy between proteins, peptides, LAB and minerals could amplify the resulting immunomodulatory effects.

MPM can be used under a humid form or dried and can be lyophilized or dried by other means and once dried, the MPMs are also compressible with a Carver press to form solid tablets. Lyophilized MPMs are compressible without the need to add any excipients to form tablets that could have multiple applications like incorporation of probiotics or drugs. MPM can integrate water, oil or other solvent to improve its general properties. MPM represent an inexpensive product with a variety of competitive advantages and applications.

Several drugs may be formulated with the MPM and they may be delivered orally and topically. A plurality of pharmaceutically related products and drugs or bioactive materials can be formulated with the MPM like small molecules of various classes (hydrophilic and hydrophobic), proteins, RNA, oligonucleotides, DNA, viruses and bacteria.

Suitable bioactive materials also include therapeutic and prophylactic agents. These include, but are not limited to any therapeutically effective biological modifier. Such modifiers include, but are not limited to lipids, organics, proteins and peptides (synthetic and natural), peptide mimetics, hormones (peptides, steroid and corticosteroid), D and L amino acid polymers, oligosaccharides, polysaccharides, nucleotides, oligonucleotides and nucleic acids, including DNA and RNA, protein nucleic acid hybrids, small molecules and physiologically active analogs thereof. Further, the modifiers may be derived from natural sources or made by recombinant or synthetic means and include analogs, agonists and homologs. As used herein "protein" refers also to peptides and polypeptides. Such proteins include, but are not limited to enzymes, biopharmaceuticals, growth hormones, growth factors, insulin, monoclonal antibodies, interferons, interleukins and cytokines.

The present disclosure describes a method of immunomodulating an immune response in a human subject comprising administering to a subject, in a preventive or therapeutic approach, a malleable protein matrix in an amount effective to modulate the biological activity of Th₁₇ cells. The immune response is for example an inflammatory response including allergy, arthritis, psoriasis, and inflammatory bowel disease. Alternatively, the immune response is an autoimmune response, including multiple sclerosis, uveitis, systemic lupus erythematosus and diabetes. In another embodiment, the immune response is a response to a cancer, a metaflammatory response including obesity, insulin resistance and type 2 diabetes, a wound healing or a response to a bacterial, viral or fungal infection. The immune response can also be an augmentation of the natural defenses or the diminution of the inflammatory processes in a preventive approach.

Also contemplated herein is the administering of an additional immunosuppressive or anti-inflammatory agent, concurrently or prior to/subsequent to the administration of MPM, within the same or different dosage.

In various embodiments, the disclosure relates to medicaments, dietary supplements, functional food, cosmeceutical supplements and medical food.

The terms activation, stimulation and treatment, as used herein and applied to cells or to receptors, may have the same meaning, e.g., activation, stimulation, or treatment of a cell or receptor with a ligand, unless indicated otherwise by the context or explicitly. The term ligand encompasses natural and synthetic ligands, e.g., cytokines, cytokine variants, analogues, muteins, and binding compositions derived from antibodies. Also encompass are small molecules, e.g., peptide mimetics of cytokines and peptide mimetics of antibodies. The expression activation can also refer to cell activation as regulated by internal mechanisms as well as by external or environmental factors. A response, e.g., of a cell, tissue, organ, or organism, encompasses a change in biochemical or physiological behavior, e.g., concentration, density, adhesion, or migration within a biological compartment, rate of gene expression, or state of differentiation, where the change is correlated with activation, stimulation, or treatment, or with internal mechanisms such as genetic programming.

The activity of a molecule describe or refer to the binding of the molecule to a ligand or to a receptor, to catalytic activity; to the ability to stimulate gene expression or cell signaling, differentiation, or maturation; to antigenic activity, to the modulation of activities of other molecules, and the like. The activity of a molecule also refer to the activity of modulating or maintaining cell-to-cell interactions, e.g., adhesion, or the activity of maintaining a structure of a cell, e.g., cell membranes or cytoskeleton. The term activity can also mean specific activity, e.g., [catalytic activity]/[mg protein], or [immunological activity]/[mg protein], concentration in a biological compartment, or the like. A proliferative activity encompasses an activity that promotes, that is necessary for, or that is specifically associated with, e.g., normal cell division, as well as cancer, tumors, dysplasia, cell transformation, metastasis and angiogenesis.

Administration and treatment, as it applies to an animal, human, experimental subject, cell, tissue, organ, or biological fluid, refers to contact of an exogenous pharmaceutical, therapeutic, diagnostic agent, or composition to the animal, human, subject, cell, tissue, organ, or biological fluid. Administration and treatment can refer, e.g., to therapeutic, pharmacokinetic, diagnostic, research, and experimental methods. Treatment of a cell encompasses contact of a reagent to the cell, as well as contact of a reagent to a fluid, where the fluid is in contact with the cell. Such administration and treatment also means *in vitro* and *ex vivo* treatments, e.g., of a cell, by a reagent, diagnostic, binding composition, or by another cell. A treatment, as it applies to a human, veterinary, or research subject, refers to therapeutic treatment, prophylactic or preventative measures, to research and diagnostic applications. Further, a treatment as it applies to a human, veterinary, or research subject, or cell, tissue, or organ, encompasses for example the contact of a Th₁₇ cells modulator to a human or animal subject, a cell, tissue, physiological compartment, or physiological fluid.

As intended herein, an immunomodulation is the process of modifying an immune response caused by agents that stimulate or reduce its function. Encompassed herein is the improvement of the immunoresponse or decrease in health problems associated with an inflammatory reaction. For example, the increased production of IL-23 in healthy individuals stimulates Th₁₇ cells to produce IL-17 and consequently activates the granulocyte-dependent response. Moreover, for an individual in a chronic inflammatory state, a lower level or the prevention of any subsequent increase of the level of IL-23 will reduce or control the development of the inflammatory reaction.

An effective amount, as defined herein, encompasses an amount sufficient to ameliorate or prevent a symptom or sign of the medical condition. Effective amount also means an amount sufficient to allow or facilitate diagnosis. An effective amount for a particular patient or veterinary subject may vary depending on factors such as the condition being treated, the overall health of the patient, the method route and dose of administration and the severity of side affects. An effective amount can be the maximal dose or dosing protocol that avoids significant side effects or toxic effects. The effect will result in an improvement of a diagnostic measure or parameter by at least 5%, usually by at least 10%, more usually at least 20%, most usually at least 30%, preferably at least 40%, more preferably at least 50%, most preferably at least 60%, ideally at least 70%, more ideally at least 80%, and most ideally at least 90%, where 100% is defined as the diagnostic parameter shown by a normal subject.

Immune condition or immune disorder encompasses, e.g., pathological inflammation, an inflammatory disorder, and an autoimmune disorder or disease. Immune condition also refers to infections, persistent infections, and proliferative conditions, such as cancer, tumors, and angiogenesis, including infections, tumors and cancers that resist eradication by the immune system. Cancerous condition includes, e.g., cancer, cancer cells, tumors, angiogenesis, and precancerous conditions such as dysplasia.

Inflammatory disorder means a disorder or pathological condition where the pathology results, in whole or in part, from, e.g., a change in number, change in rate of migration, or change in activation, of cells of the immune system. Cells of the immune system include, e.g., T cells, B cells, monocytes or macrophages, antigen presenting cells (APCs), dendritic cells, microglia, NK cells, NKT cells, neutrophils, eosinophils, mast cells, or any other cell specifically associated with the immunology, for example, cytokine-producing endothelial or epithelial cells.

Metaflammation means a metabolically triggered inflammation, sometimes referred to as "low-grade" or "chronic" inflammation. This condition is triggered by nutrients, metabolic surplus or aging, and engages a similar set of molecules and signaling pathways to those involved in classical inflammation. Obesity, insulin resistance, type 2 diabetes, hypertension, fatty liver disease, atherosclerosis, degenerative disorders, airway disease and some cancer are closely associated with metaflammation.

Other pathologic conditions can produce a chronic inflammation and induce several metabolic disorders like in people with AIDS. HIV infection results in the adhesion of the gp120 protein with the CD4 molecule on immune cells and necessitate also the adhesion of co-stimulatory molecule such as the *β*-chemokine CCR5 and the *α*-chemokine CXCR4. The principal immune cells that permits the development of virus is CD4+ T cells but also dendritics cells and monocytes/macrophages, which possess also surface CD4 molecules in a small amount. The development of AIDS in HIV-positive patients will occur in an average of 10 years. During this period of time, the replication of virus is markedly increase and is associated with the loss of immune functionality. Moreover, the HIV replication creates a constant inflammatory status in patients. The death occurs in approximately 12 years and the incidence of death resulting to AIDS and its complications is more than 3 million annually.

The more important complication of AIDS is the suppressive effect on immunity, and principally, the decrease in CD4+ T cells number and in the functionality of its T-helper role to communicate with other white blood cells in immune defenses. Firstly, the virus infects and replicates itself in CD4+ T cells causing the destruction of these cells. Secondly, the major cause of CD4+ T cells destruction is the association of HIV on CD4 receptors which leads to apoptosis of these CD4 cells. Another important immune deregulation of HIV-infected patients is an increase in B-cells hyperactivity resulting in a higher nonspecific circulating immunoglobulin that leads to a difficulty in developing a specific antibody response when challenged. Moreover, the medication of HIV-infected patients leads to a general immunosuppression. All these immunity complications of AIDS result in an increase susceptibility to infections.

An important number of HIV-infected patients suffered of wasting syndrome causing an important weight loss, principally a muscle mass loss, associated with diarrhea, fever and fatigue. This wasting syndrome is an AIDS- related complication leading to an important rate of mortality. This wasting syndrome is a secondary cause of important production of inflammatory cytokines IL-1*β*, IL-6 and TNF-*α* by macrophages, which is demonstrated in animal model that causing wasting syndrome.

Lipodystrophy is associated with metabolic disorders such as hyperlipidemia and insulin resistance as well as an accumulation of fat in the abdomen. This complication is mostly associated with the protease inhibitors (AIDS medication) that interfere with proteolysis of transcription factors implicated in lipids homeostasis. Lipodystrophy complications lead to an adipose tissue disorder characterized by a selective loss of body fat. Patients with lipodystrophy have a tendency to develop insulin resistance, type 2 diabetes, a high blood triglyceride level and fatty liver.

Modulation of the biological activity of Th₁₇ cells can be used to improve the situation of people with AIDS by increasing polymorphonuclear cells and CD4+ population, ensuring a better response against invading pathogen, and reducing the risk of infections in AIDS immunosuppressed patients. Th17 cells modulation can also reduce inflammatory of cytokines, particularly IL-1*β*, IL-6 and TNF*α* by macrophages, responsible of monitoring the constant inflammatory status in HIV-patients and leading to many AIDS complications such as wasting syndrome, lipodystrohy, deregulation of immunity, and of propagating the virus. This inhibition of inflammatory cytokines is essential for maintaining immune homeostasis and thus, reducing AIDS complications like hyperlipidemia and weight control.

An IL-17-producing cell means a T cell that is not a classical TH₁-type T cell or classical TH₂-type T cell, referred to as TH₁₇ cells.

Moreover, IL-17-producing cell includes a progenitor or precursor cell that is committed, in a pathway of cell development or cell differentiation, to differentiating into an IL-17-producing cell, as defined above. A progenitor or precursor cell to the IL-17 producing cell can be found in a draining lymph node (DLN). Additionally, IL-17-producing cell encompasses an IL-17-producing cell, as defined above, that has been, e.g., activated, e.g., by a phorbol ester, ionophore, and/or carcinogen, further differentiated, stored, frozen, desiccated, inactivated, partially degraded, e.g., by apoptosis, proteolysis, or lipid oxidation, or modified, e.g., by recombinant technology.

An inhibitors", "antagonists" or "activators" and "agonists" refer to inhibitory or activating molecules, respectively, e.g., for the activation of, e.g., a ligand, receptor, cofactor, a gene, cell, tissue or organ. A modulator of, e.g., a gene, a receptor, a ligand or a cell, is a molecule that alters an activity of the gene, receptor, ligand or cell, where the activity can be activated, inhibited or altered in its regulatory properties. The modulator may act alone or it may use a cofactor, e.g., a protein, metal ion or small molecule. Inhibitors are compounds that decrease, block, prevent, delay activation, inactivate, desensitize or down regulate, e.g., a gene, protein, ligand, receptor or cell.

An "activators" are compounds that increase, activate, facilitate, enhance activation, sensitize or up regulate, e.g., a gene, protein, ligand, receptor or cell. An "inhibitor" may also be defined as a composition that reduces, blocks or inactivates a constitutive activity. An "agonist" is a compound that interacts with a target to cause or promote an increase in the activation of the target. An "antagonist" is a compound that opposes the actions of an agonist. An antagonist prevents, reduces, inhibits or neutralizes the activity of an agonist. An antagonist can also prevent, inhibit or reduce constitutive activity of a target, e.g., a target receptor, even where there is no identified agonist.

An immunosuppressive agents, immunosuppressive drugs, or immunosuppressant as used herein are therapeutics that are used in immunosuppressive therapy to inhibit or prevent activity of the immune system. Clinically they are used to prevent the rejection of transplanted organs and tissues (e.g. bone marrow, heart, kidney, liver), and/or in the treatment of autoimmune diseases or diseases that are most likely of autoimmune origin

(e.g. rheumatoid arthritis, myasthenia gravis, systemic lupus erythematosus, ulcerative colitis, multiple sclerosis). Immunosuppressive drugs can be classified into four groups: glucocorticoids cytostatics; antibodies (including Biological Response Modifiers or DMARDs); drugs acting on immunophilins; other drugs, including known chemotherapeutic agents used in the treatment of proliferative disorders.

The expression "anti-inflammatory agents" or "anti-inflammatory drug" are used to represent both steroidal and non-steroidal therapeutics. Steroids, also known as corticosteroids, are drugs that closely resemble cortisol, a hormone produced naturally by adrenal glands. Steroids are used as the main treatment for certain inflammatory conditions, such as: Systemic vasculitis (inflammation of blood vessels); and Myositis (inflammation of muscle). Steroids might also be used selectively to treat inflammatory conditions such as: rheumatoid arthritis (chronic inflammatory arthritis occurring in joints on both sides of the body); systemic lupus erythematosus (a generalized disease caused by abnormal immune system function); Sjogren's syndrome (chronic disorder that causes dry eyes and a dry mouth).

Non-steroidal anti-inflammatory drugs, usually abbreviated to NSAIDs, are drugs with analgesic, antipyretic and anti-inflammatory effects and they reduce pain, fever and inflammation. The term "non-steroidal" is used to distinguish these drugs from steroids, which (amongst a broad range of other effects) have a similar eicosanoid-depressing, anti-inflammatory action. NSAIDs are generally indicated for the symptomatic relief of the following conditions: rheumatoid arthritis; osteoarthritis; inflammatory arthropathies (e.g. ankylosing spondylitis, psoriatic arthritis, Reiter's syndrome); acute gout; dysmenorrhoea; metastatic bone pain; headache and migraine; postoperative pain; mild-to-moderate pain due to inflammation and tissue injury; pyrexia; and renal colic. NSAIDs include salicylates, arlyalknoic acids, 2-arylpropionic acids (profens), N-arylanthranilic acids (fenamic acids), oxicams, coxibs and sulphonanilides.

### Pharmaceutical compositions

To prepare pharmaceutical or sterile compositions, the MPM is admixed with a pharmaceutically acceptable carrier or excipient.

Formulations of therapeutic and diagnostic agents may be prepared by mixing with physiologically acceptable carriers, excipients, or stabilizers in the form of, e.g., lyophilized powders, slurries, aqueous solutions or suspensions.

Toxicity and therapeutic efficacy of the MPM, administered alone or in combination with an immunosuppressive agent, can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio between LD₅₀ and ED₅₀. The data obtained from these cell culture assays and animal studies can be used in formulating a range of dosage for use in human. The dosage of such compounds lies preferably within a range of circulating concentrations that include the ED₅₀ with little or no toxicity. The dosage may vary within this range depending upon the dosage form employed and the route of administration utilized.

Oral administration to the individual is preferred. Other suitable routes of administration may, for example, include rectal, cutaneous, or intestinal administration.

Determination of the appropriate dose is made by the clinician, e.g., using parameters or factors known or suspected in the art to affect treatment or predicted to affect treatment. Generally, the dose begins with an amount somewhat less than the optimum dose and it is increased by small increments thereafter until the desired or optimum effect is achieved relative to any negative side effects. Important diagnostic measures include those of symptoms of, e.g., the inflammation or level of inflammatory cytokines produced. Preferably, a biologic that will be used is derived from the same species as the animal targeted for treatment, thereby minimizing an inflammatory, autoimmune, or proliferative response to the reagent.

As used herein, the term "treat" or "treatment" includes a postponement of development of the symptoms associated with autoimmune disease or pathogen-induced immunopathology and/or a reduction in the severity of such symptoms that will or are expected to develop. The terms further include ameliorating existing uncontrolled or unwanted autoimmune-related or pathogen-induced immunopathology symptoms, preventing additional symptoms, and ameliorating or preventing the underlying causes of such symptoms. Thus, the terms denote that a beneficial result has been conferred on a vertebrate subject with an autoimmune or pathogen-induced immunopathology disease or symptom, or with the potential to develop such a disease or symptom.

As used herein, the term "therapeutically effective amount" or "effective amount" refers to an amount of MPM, that when administered alone or in combination with an additional therapeutic agent to a cell, tissue, or subject is effective to immunomodulate, prevent or ameliorate the autoimmune disease or pathogen-induced immunopathology associated disease or condition or the progression of the disease. A therapeutically effective dose further refers to that amount of the compound sufficient to result in amelioration of symptoms, e.g., treatment, healing, prevention or amelioration of the relevant medical condition, or an increase in rate of treatment, healing, prevention or amelioration of such conditions. When applied to an individual active ingredient administered alone, a therapeutically effective dose refers to that ingredient alone. When applied to a combination, a therapeutically effective dose refers to combined amounts of the active ingredients that result in the therapeutic effect, whether administered in combination, serially or simultaneously. An effective amount of therapeutic will decrease the symptoms typically by at least 10%; usually by at least 20%; preferably at least about 30%; more preferably at least 40%, and most preferably by at least 50%.

Methods for co-administration or treatment with a second therapeutic agent (concurrently or prior to/subsequent to administering the pharmaceutical composition described herein), e.g., a cytokine, steroid, chemotherapeutic agent, antibiotic, or radiation, are well known in the art. The pharmaceutical composition of the present disclosure may also contain other immunosuppressive or immunomodulating agents. Any suitable immunosuppressive agent can be employed, including but not limited to anti-inflammatory agents, corticosteroids, cyclosporine, tacrolimus (i.e., FK-506), sirolimus, interferons, soluble cytokine receptors (e.g., sTNRF and sIL-1R), agents that neutralize cytokine activity (e.g., inflixmab, etanercept), mycophenolate mofetil, 15-deoxyspergualin, thalidomide, glatiramer, azathioprine, leflunomide, cyclophosphamide, methotrexate and the like. The pharmaceutical composition can also be employed with other therapeutic modalities such as phototherapy and radiation.

Typical veterinary, experimental, or research subjects include monkeys, dogs, cats, rats, mice, rabbits, guinea pigs, horses, and humans.

### Dietary supplement, nutraceutical/functional or medical food composition

MPM is also useful as a component of a dietary supplement, nutraceutical/functional or medical food. Dietary supplements, nutraceutical/functional or medical food suitable for use in the present invention include compositions wherein the active ingredients are contained in an effective amount to achieve its intended purpose. Determination of the effective amounts is well within the capability of those skilled in the art, especially in light of the detailed disclosure provided herein. The amount of composition administered will be dependent upon the condition being treated, the subject being treated, on the subject's weight, the severity of the affliction, the manner of administration and the judgment of the individual's physician. The ingredients of the dietary supplement of this invention are contained in acceptable excipients and/or carriers for oral consumption. The actual form of the carrier, and thus, the dietary supplement itself, may not be critical. The carrier may be a liquid, gel, gelcap, capsule, powder, solid tablet (coated or non-coated), dairy product/food product or the like. Suitable excipient and/or carriers include maltodextrin, calcium carbonate, dicalcium phosphate, tricalcium phosphate, microcrystalline cellulose, dextrose, rice flour, magnesium stearate, stearic acid, croscarmellose sodium, sodium starch glycolate, crospovidone, sucrose, vegetable gums, agar, lactose, methylcellulose, povidone, carboxymethylcellulose, corn starch, and the like (including mixtures thereof). The various ingredients and the excipient and/or carrier are mixed and formed into the desired form using conventional techniques. Dose levels/unit can be adjusted to provide the recommended levels of ingredients per day in a reasonable number of units. The dietary supplement may also contain optional ingredients including, for example, herbs, vitamins, minerals, enhancers, colorants, sweeteners, flavorants, inert ingredients, and the like. Such optional ingredients may be either naturally occurring or concentrated forms. Selection of one or several of these ingredients is a matter of formulation, design, consumer preference and end-user. The amounts of these ingredients added to the dietary supplements nutraceutical/functional or medical food of this disclosure are readily known to the skilled artisan.

### Cosmeceutical composition

MPM is also useful as a component of a cosmeceutical supplement. Cosmeceutical supplements include compositions wherein the active ingredients are contained in an effective amount to achieve its intended purpose. Such cosmeceutical supplements can be formulated for delivery by a mode selected from the group consisting of but not restricted to oral delivery, spray, injection, drops, perfusion, irrigation, topical skin application and topical application during surgery. Determination of the effective amounts is well within the capability of those skilled in the art, especially in light of the detailed disclosure provided herein. The ingredients of the cosmeceutical supplement are contained in acceptable excipients and/or carriers for dermal application. The actual form of the carrier, and thus, the cosmeceutical product itself, may not be critical. A cosmeceutical supplement exhibiting biological properties for skin care and maintenance, repair, skin regeneration, and anti-aging in products like skin care, sunscreens, which include baby creams, emollient creams, cold creams, conditioning creams, protective creams, sunscreen lotion, lip balm, lipsticks, eye shadows and bar soaps or the like. The various ingredients and the excipient and/or carrier are mixed and formed into the desired form using conventional techniques. Dose levels/unit can be adjusted to provide the recommended levels of ingredients per day in a reasonable number of units. The cosmeceutical supplement may also contain optional ingredients including, for example, herbs, vitamins, minerals, enhancers, colorants, sweeteners, flavorants, inert ingredients and the like. Such optional ingredients may be either naturally occurring or concentrated forms. Selection of one or several of these ingredients is a matter of formulation, design, consumer preference and end-user. The amounts of these ingredients added to the cosmeceutical product of this disclosure are readily known to the skilled artisan.

The present invention will be more readily understood by referring to the following examples which are given to illustrate the invention rather than to limit its scope.

### EXAMPLE 1

### Generation of MPM

MPM are obtained by fermenting sweet whey with lactic acid bacteria from the *Lactobacillus* genus, followed by a protein-specific recuperation procedure. The product is produce by Technologie Biolactis Inc. at industrial scale by fermentation of *Lactobacillus* Kefiranofaciens R2C2 strain like as described below.

The first step is a pre-culture in fermentor where frozen ferment culture, R2C2 (strain accession number: 041202-3; National Microbiology Laboratory, Health Canada, 1015 Arlington Street, Winnipeg, Manitoba, Canada, R3E 3R2), is used to inoculate pre-culture medium. The pre-culture medium is a medium composed of whey powder 6.7% (w/v), yeast extract 0.41 % (w/v) (Biospringer, 0202), yeast peptone 0.12% (w/v) (BioSpringer, Hyp A), water 92.8%. The grow media is pasteurized at 82°C (180°F) +/- 2°C for 35 minutes, then cool down at 37°C. The incubation of the strain, with a ratio of initial inoculation of 10% (10⁸ bacteria/ml), is at 37°C for 24 hours. Initial pH should be 5.3 +/- 0.3 and final ph after 24h should be 3.8 (+/-0.1).

The second step is the crude cheese whey treatment in which the whey is pasteurized to destruct its microbiological flora. The cheese whey pasteurization is conducted using heat exchanger. After pasteurization, the cheese whey is inoculated with 10% (v/v) of the pre-culture. The fermentation is realized at 37°C with controlled temperature for twelve hours. Agitation is maintained to a minimum to allow a uniform distribution but without causing an excessive aeration. The fermentation is follow-up by the addition of calcium chloride 0,3% (wt/vol) and the adjustment of the pH to 7,5 with NaOH. The fermented cheese whey is heat treated a second time using heat exchanger and the MPM recovery is achieved using a VNPX710 clarifying unit of Alpha Laval (Alfa Laval, Sweden). The consistency was adjusted to a yogurt-like by clarifying recuperation adjustment. The resulting MPM is malleable, looks like a pudding of white creamy color with no noticeable taste or smell. This MPM consists mainly (wt/wt), of water (80,3%), protein (8,6%), minerals (4,7%, of which calcium comprises 1,5%), carbohydrate (1,5%), fat (1,3%) and bacteria (6X10¹¹/100g).

After recovery, the MPM is cooled down to 4°C and packed in hermetic packaging. The MPM is stored at 4°C for 5 days to complete the microbiologic analyses. They can be uses as it is or dried.

Those skilled in the art will recognize, or be able to ascertain using no more than routine experimentation, numerous equivalents to the specific procedure described herein. Such equivalents are considered to be within the scope of this invention. The international patent application publication No WO 03/053158 discloses different way of producing MPM.

### EXAMPLE 2

### Effect of MPM on stimulation of the immune pathways in healthy animals

Water (100µl; control) or MPM (1 mg/g) was administered *p*.*o*. twice a day for 4 days. After this period, the lymphocyte, monocyte and polymorphonuclear cells were identified with biotin anti-rat CD45 specific marker and streptavidin-PE-Cy5 to complete the reaction. The red blood cells were lysed with Optilyse C™ in accordance with manufacturer's instructions. The cell counts were obtained by passage of 20 *µ*l of preparation in a Flow Cytometry Epics XL™ *(Beckman Coulter, Fullerton).* The lymphocytes, monocytes and polymorphonuclears were separated in accordance with size and surface expression level of molecule CD45.

In addition, water (100µl) or MPM (1 mg/g) was administered p.o. each day in the morning for 2 weeks. After this period, the mice were sacrificed with CO₂ asphyxia and their spleens were kept in PBS supplemented with protease inhibitors at -80°C. Spleens were homogenized and clarified by centrifugation for 15 minutes at 1500Xg to remove excess connective tissue and large aggregate debris. The supernatant was then removed from the cellular debris pellet and stored frozen at -80°C until analyzed for cytokine content. The cytokine profile was determined with the Searchlight Multiplex Assays sample testing service *(Endogen, Woburn, USA).*

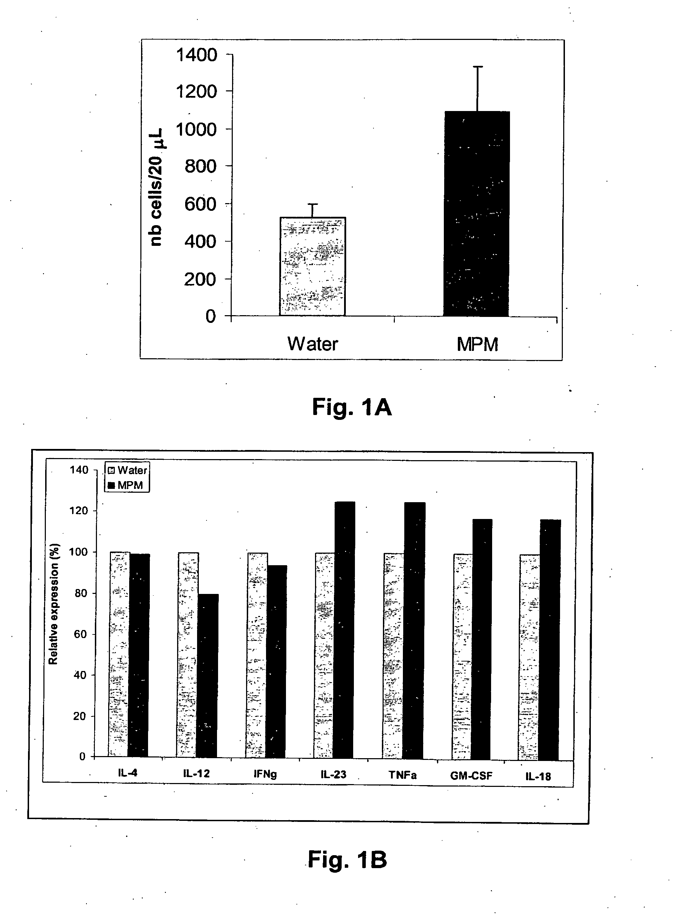

In healthy animals, a significant increase of blood PMN cells was observed following MPM administration (Fig. 1A) indicating an immune response. The cytokine production profile demonstrates that the Th₂ pathway is not affected as seen by the absence of IL-4 production (Fig. 1B), the principal Th₂ pathway cytokine. The Th₁ pathway is activated by an increase of IL-12 and consequently, IFN*γ* production. The MPM reduces the production of IL-12 as well as the resulting cytokine, the IFNγ (Fig. 1B). These results demonstrate that the stimulation of immunity by the MPM consumption is not related to the Th₁ pathway. The Th₁₇ pathway is activated by an increase in IL-23 production and consequently, the production of TNF*α* and GM-CSF will increase (Iwakura et al., 2006, J. Clin. Inves. 116: 1218-1222). The GM-CSF production is responsible of the differentiation of PMN cells via bone marrow. The MPM activated the production of all these cytokines, IL-23, .TNF*α* and GM-CSF (Fig 1 B). These results explain the observed increase of PMN cells following MPM administration (Fig. 1A). It is known that IL-18 is implicated in Th₁ development if this cytokine is in presence of IL-12 but in presence of TNF*α* and IL-23, this synergy will increase the production of Th₁₇ cells (Nakae et al., 2007, J Leukoc Biol, 81: 1258-1268). The MPM reduces IL-12 production but increase IL-23, TNF*α* and IL-18 (Fig. 1B) indicating a stimulation of Th₁₇ pathway but not Th₁ pathway.

The MPM stimulates immunity through Th₁₇ pathway and consequently, activates an anti-infectious defense via the granulocyte-dependent response.

### EXAMPLE 3

### Effect of MPM on systemic cytokine profile in an Atopic Contact Dermatitis (ACD) model.

Water (100µl; control) or MPM (1 mg/g) were administered *p.o.* each day in the morning for 1 week. After this period, the murine model of ACD was done as follows: abdomen hairs of CD-1 mice were removed and the sensitization phase was done with the application of 100 *µ*L of oxazolone 5 % in acetone on the hairless abdomen. After 4 days, the elicitation phase (first challenge) was initiated with application of 50 *µ*l of oxazolone 5% in acetone on the right ear (25 *µ*l each side of the ear). The second challenge was done 7 days after the first challenge with the same procedure. The water or MPM was administered *per os* each day during the entire experiment. After that, the mice were sacrificed with CO₂ asphyxia and their spleens were kept in PBS supplemented with protease inhibitors at -80°C. Treated ears were snap frozen in liquid nitrogen and stored at -80°C. Spleens were homogenized and clarified by centrifugation for 15 minutes at 1500Xg to remove excess connective tissue and large aggregate debris. The supernatant was then removed from the cellular debris pellet and stored frozen at -80°C until analyzed for cytokine content. The cytokine profile was determined with the Searchlight Multiplex Assays sample testing service *(Endogen, Woburn, USA).* Total RNA from ears was extracted using Trizol reagent (Life Technologies) according to the manufacturer's instructions. The RNA purity was evaluated by spectrophotometry. Total RNA (1 µg) from ears was added to a 20-µl reverse transcription-PCR reaction using RT² First Strand kit (SABiosciences)." Each real-time PCR was performed on a ABI 7900HT FAST 96-well blocks on a cDNA pooled of four mice (treated or not with MPM) according to the manufacturer's manual instructions (Mouse Th17 for Autoimmunity and Inflammation RT² Profiler PCR Arrays, Type C, SABiosciences). The PCR results are representative of a population of mice treated or not with MPM.

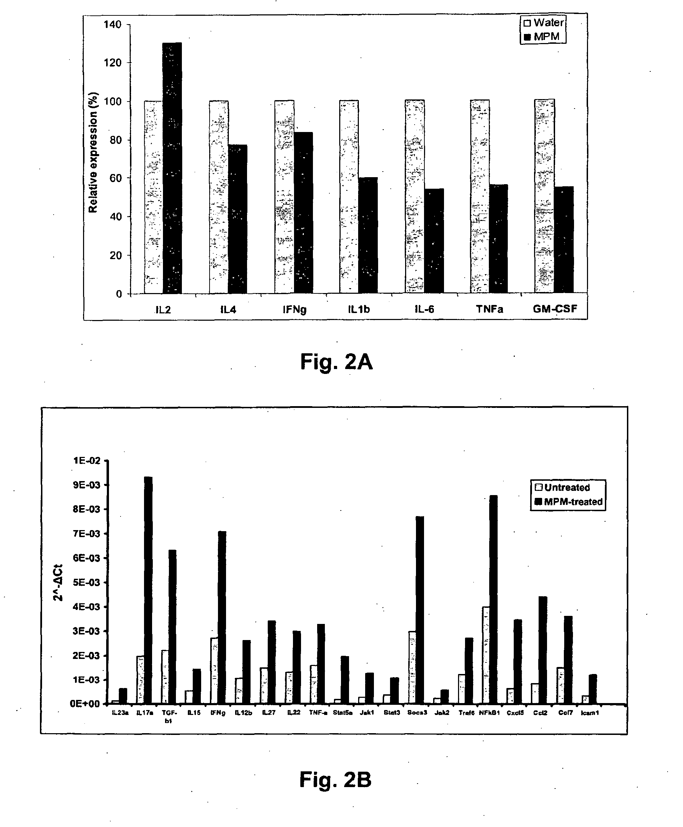

In the atopic contact dermatitis model, the inflammation is the consequence of activation of Th1 pathway as well as Th₁₇ pathway. The inflammation in a tissue created by Th₁ cells is particularly due to IFNγ production and the inflammation created by Th₁₇ cells is the consequence of TNF*α*, IL-1*β* and IL-6 production by resident cells. The administration of MPM does not increase the IL-4 production; on the contrary, IL-4 production is lower indicating that MPM does not possess the potential to regulate the inflammation via increases in Th₂\Th₁ ratio (Fig. 2A). Moreover, the IFN*γ* is not significantly reduced following MPM consumption. A mild reduction of IFN*γ* production is observed but this reduction cannot explain the anti-inflammatory effect observed. This mild decrease in IFN*γ* production is a consequence of the reduction of inflammation that consequently, inhibits all inflammatory cytokine production. The inhibition of Th₁ pathway is not the mechanism of action of anti-inflammatory effect of MPM. The production of cytokines TNF*α*, IL-1*β*, IL-6 and GM-CSF is lower following MPM consumption (Fig. 2A). These results indicate that MPM modulates the Th₁₇ pathway. Moreover, the production of IL-2 is higher following MPM administration. It is known that IL-2 inhibits the expansion and survival of Th₁₇ cells as well as the IL-17 production by activated Th₁₇ cells (Laurence et al., 2007, Immunity, 26: 371-381). The administration of MPM increases the IL-2 production and consequently, modulates Th₁₇ cells. At the transcriptional level at the inflammation site (ear), MPM treatment clearly modulates the expression of IL-17a and other genes involved in the Th-17 pathway (Fig. 2B), suggesting a control at the transcriptional and post-transcriptional level; a balance between genes expression and cytokines production and survival.

Consequently, MPM significantly reduces the inflammation in this model and this anti-inflammatory effect is comparable to those obtained with an oral hydrocortisone treatment without the immunosuppression observed following hydrocortisone administration (Beaulieu et al., 2007, J. Inflamm. 4: 6).

All these results indicate that the mechanism of action of anti-inflammatory effect of MPM is related to the modulation of Th₁₇ pathway at the transcriptional and post-transcriptional level.

### EXAMPLE 4

### Effect of MPM on local cytokine profile in a rheumatoid arthritis-like model: the air pouch model.

Water (100µl; control) or MPM (1 mg/g) was administered p.o. each day in the morning for 2 weeks. The mechanism implicated in the inflammation in the air pouch model is similar than that observed in the rheumatoid arthritis. Briefly, at day 8 and 11, mice were anesthetized with isoflurane and 3.0 cc of sterile air passed through a 0.22 *µ*m filter mounted to a syringe was injected subcutaneously with a 26-gauge needle in the back of mice to created the air pouch. At day 14, 1 ml of lipopolysaccharide (1 *µ*g/ml) was injected into the air pouch. After an incubation period of 6 hours, the infiltrated cells were washed once with 1 ml of HBSS (Hank's balanced salt solution) and then twice with 2 ml of HBSS, 10 mM EDTA (ethylenediaminetetraacetic acid). The cells were pooled, washed twice in PBS and counted on Confocal microscopy.

The supernatant of the first wash was stored frozen at -80°C until analyzed for the detection of cytokines. The cytokine profile was determined with the Searchlight Multiplex Assays sample testing service *(Endogen, Woburn, USA).*

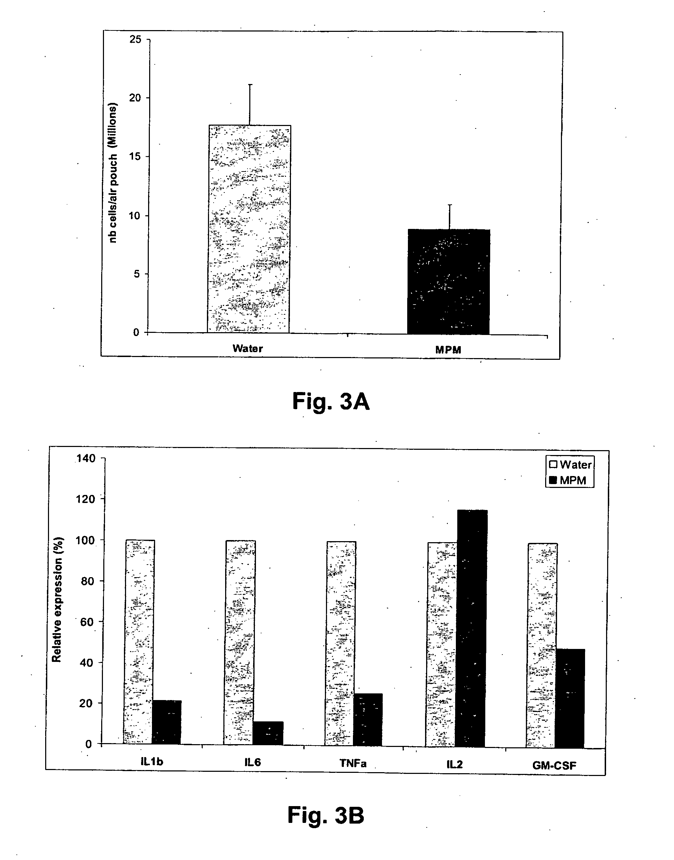

An important inhibition of neutrophil infiltration in the air pouch model is observed following MPM administration (Fig. 3A). This inhibition of neutrophil infiltration is associated with a lower production of cytokines IL-1*β*, IL-6 TNF*α* and GM-CSF (Fig. 3B). These cytokines are produced following activation of Th₁₇ cells indicating that the inhibition of their production by MPM is related to modulation of the Th₁₇ pathway. Moreover, the increase in IL-2 production following MPM consumption is responsible of the inhibition of the expansion and survival of Th₁₇ cells. All these results indicate that the mechanism of action of anti-inflammatory effect of MPM is related to the modulation of Th₁₇ pathway.

### EXAMPLE 5

### Effect of MPM on IL-23 production in a mild inflammatory animal model.

Water (100µl; control) or MPM (1 mg/g) was administered p.o. each day in the morning for 2 weeks. After that period, 1 ml of proteose peptone 3% was injected intra-peritoneal. Four days after the injection, mice were sacrificed with CO₂ asphyxia and their spleen was kept in PBS supplemented with protease inhibitors at -80°C. Spleens were homogenated and clarified by centrifugation for 15 minutes at 1500Xg to remove excess connective tissue and large aggregate debris. The supernatant was then removed from the cellular debris pellet and stored frozen at -80°C until analyzed for cytokine content. The IL-23 production was determined with the Searchlight Multiplex Assays sample testing service *(Endogen, Woburn, USA).*

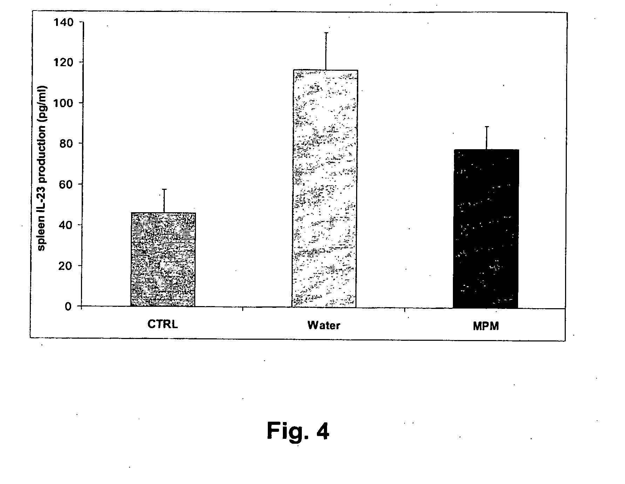

The IL-23 production is crucial in the development of Th₁₇ pathway. The systemic production of IL-23 is 50% lower in the MPM-treated group in comparison with both non-inflammatory control and water-treated group (Fig. 4). This 50% reduction of IL-23 production explains the important anti-inflammatory effect of MPM and confirms that the mechanism of action of MPM is related to regulation of Th₁₇ pathway.

### EXAMPLE 6

### Effect of MPM on weight management and body composition

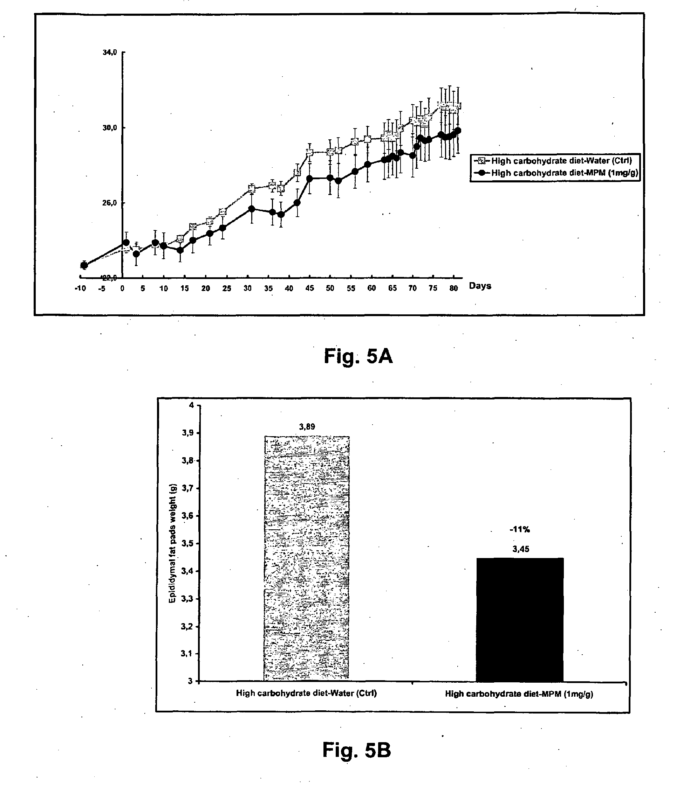

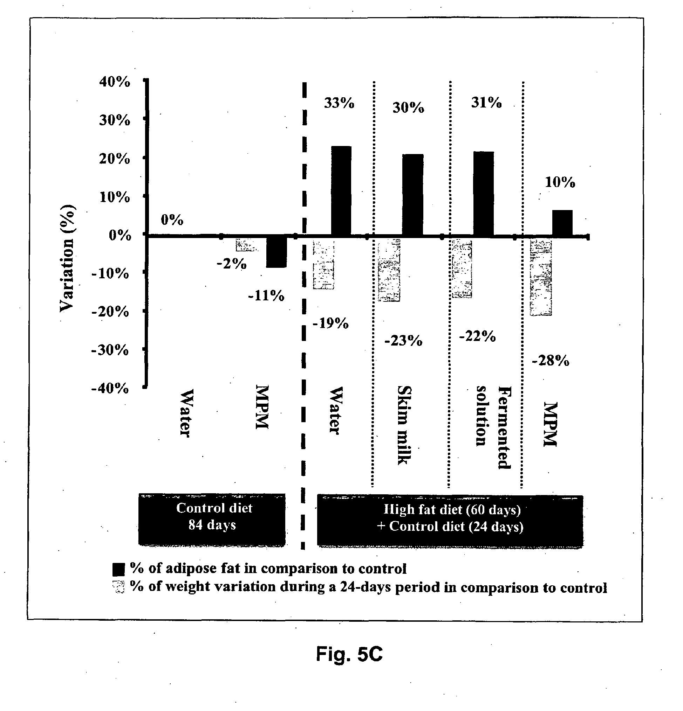

In a first experiment (Fig. 5A, 5B), male C57BI/6J mice were put on a high carbohydrate diet (48% dextrin, 19% sucrose). Treatments consisted of daily intragastric gavage (5 days a week) with water (control group, 100µl) or MPM (1 mg/g). In the second experiment (Fig 5C), male C57BI/6J mice were put on a high fat diet (34,7% fat) followed by a weight lost period on a control diet (5,2% fat) for 24 days. Treatments consisted of daily intragastric gavage (5 days a week) with water (control group, 100µl), skim milk (100µl), fermented solution (100µl) or MPM (1mg/g). In both experiments, animals were maintained in a 12-hour light/dark cycle and consumed their diet and water *ad libitum* for 12 weeks. Animal weights were recorded daily (Fig 5A). At the end of the experiment, animals were sacrificed and epididymal fat pads weighted (Fig 5B and 5C).

As demonstrated, MPM has an effect on obesity and weight management. Animals fed on a high carbohydrate diet with MPM were leaner (-6%) than water-treated counter-part. It appears also that lower fat was produce in the MPM-treated group as shown in Figure 5B (-11%). The ratio of epididymal fat weight/mice weight demonstrated that the body composition of mice differs from MPM-treated mice and water-treated mice. Figure 5B and 5C demonstrates that MPM reduces the fat content and modifies the body composition (Fig. 5B and Fig. 5C).

Most adipose tissue in mammals is white adipose tissue. It contains adipocytes, pre-adipocytes (not loaded with lipids), endothelial cells, fibroblasts, leukocytes and macrophages. These macrophages are derived from the bone-marrow and their numbers correlates with apparition of obesity. Expansion of the adipose tissue during weight gain leads to the recruitment of macrophages by adipocytes. Various mediators synthesized by adipocytes as well as macrophages (such as IL-1, IL-6, TNF-α and CCL2) contribute to local and systemic inflammation favoring a pro-inflammatory milieu (Tilg et al., 2006, Nature Rev 6: 772-783).

The MPM prevent obesity through the Th₁₇ pathway by negatively regulating the granulocyte-dependent response and thus preventing recruitment of immune cells like macrophages in adipose tissue. MPM will down regulate the production of pro-inflammatory cytokines like IL-6, TNF-*α* and CCL2, impairing the crosstalk between with adipocytes and marcophages and preventing lean adipose tissue to become obese adipose tissue (Tilg et al., 2006, Nature Rev 6: 772-783). Indeed, obesity is considered as a metaflammation state in which the plasma concentration of cytokines IL-1*β*, IL-6 and TNF-*α* are increased due to elevated production of these mediators by adipocytes. The production of cytokines TNF*α*, IL-1*β*, IL-6 and GM-CSF significantly decreases following MPM consumption (Fig. 2A). MPM has demonstrated an anti-inflammatory effect associated with a reduction of these specific cytokines indicating an inhibition of the inflammatory state associated to obesity.

### EXAMPLE 7

### Effect of MPM on total plasma triglycerides and cholesterol level in a poloxamer-induced hyperlipidemia rat model.

Female Wistar rats were randomly assigned to various treatment groups. Animals were maintained in a 12-hour light/dark cycle and consumed standard diet and water *ad libitum.* Rats were pretreated by intragastric gavages daily for 7 days with water (placebo group, 1 ml), MPM (200 mg/day) or niacin (25 mg/day) which is known to exert a positive effect on triglycerides and cholesterol metabolism in this model. Following 7 days pretreatment, all animals were rendered hyperlipidemic by an *i.p.* injection of 300 mg of poloxamer 407 (P407, BASF Corporation). Daily intragastric gavages were continued for 3 days (72h) during the P407-induced hyperlipidemic state. Blood samples were collected for determination of total plasma cholesterol, triglycerides at 72h post-induction of hyperlipidemia. All blood lipid analyses were performed in an independent laboratory.

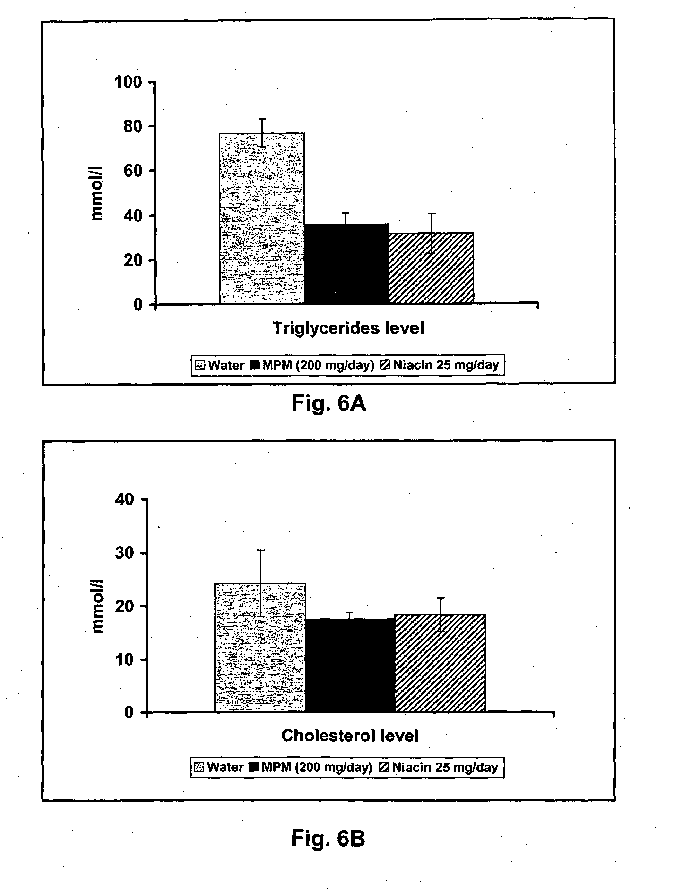

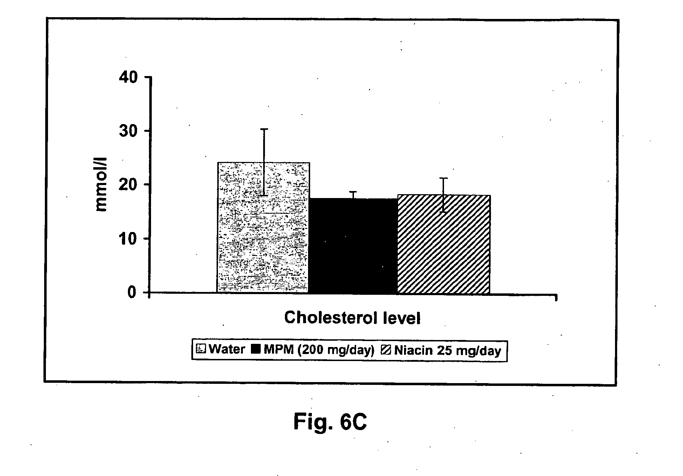

As seen in figures 6A, 6B and 6C, MPM (200mg/day) is able to reduce plasma triglycerides and cholesterol levels (-51% and -26% respectively) at 72 hours after hyperlipidemia compare to water-treated animals. These results on hyperlipidemia suggested a beneficial impact on plasma lipid levels associated with consumption of MPM. MPM showed the capacity to regulate lipid levels in laboratory animals at basal state and in the poloxamer 407-induced hyperlipidemia model. In every aspect tested, MPM seemed to have lipid-lowering properties as good as niacin. This model is also used to measure the impact on atherosclerosis.

The production of cytokines TNF*α*, IL-1*β*, IL-6 and GM-CSF significantly decreases following MPM consumption (Fig. 2A). The MPM prevent hyperlipidemia through the Th₁₇ pathway by negatively regulating the granulocyte-dependent response and thus preventing recruitment of immune cells from the bone-marrow like macrophages in adipose tissue. These will down regulate the production of pro-inflammatory cytokines like IL-6, TNF*α* and CCL2, impairing the crosstalk between with adipocytes and marcophages and thus preventing pre-adipocyte to become fully mature lipid-charged adipocyte. MPM has an anti-inflammatory effect associated with a reduction of Th₁₇ cells specific cytokines indicating an inhibition of vascular endothelial dysfunction, an inhibition of abnormal lipid profile, hypertension and vascular inflammation which promote the development of atherosclerotic cardiovascular disease.

### EXAMPLE 8

### Effect of MPM on the fasting blood glucose tolerance test (OGTT) of rats on a high fructose diet.

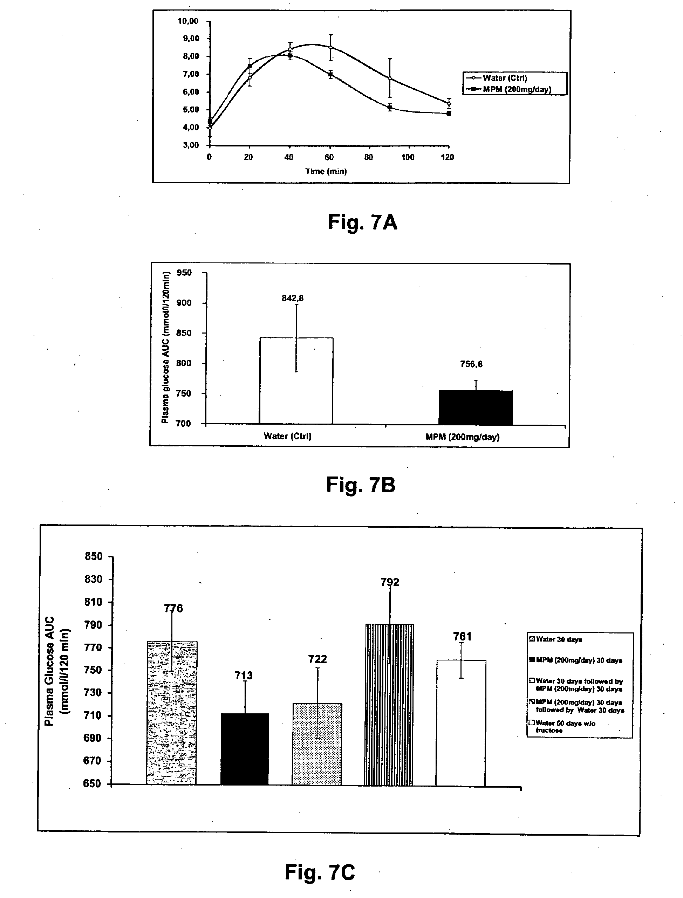

Male Wistar rats were put on a high fructose diet (10%) for two weeks. The treatments consisted of daily intragastric gavages (7 days a week) with either water (placebo group, 1 ml), or MPM (200mg/day). Animals were maintained in a 12-hour light/dark cycle and consumed their diet and water ad *libitum* for 15 days. On day 15, overnight fasted animals from each group were subjected to oral glucose tolerance test (oral glucose load 2g/kg). Thereafter, following oral glucose load, blood samples were collected at 0, 20, 40, 60, 90 and 120 min and glucose was measured using the FreeStyle™ mini apparatus from ThermaSense. The area under the curve (Fig. 7A) was then calculated to evaluate glucose sensitivity (Fig. 7B). Also, male Wistar rats were put on a high fructose diet (10%) for 30 days or normal diet (water control without fructose). The treatments consisted of daily intragastric gavages (7 days a week) with either water (1 ml), or MPM (200mg/day). After 30 days, overnight fasted animals from each group were subjected to oral glucose tolerance test (oral glucose load 2g/kg). Thereafter, following oral glucose load, blood samples were collected at 0, 20, 40, 60, 90 and 120 min and glucose was measured using the FreeStyle™ mini apparatus from ThermaSense. The area under the curve was then calculated to evaluate glucose sensitivity. Animals on water were then switch to MPM and vice versa (Fig. 7C).

Experiments on Wistar rats treated or not with MPM suggested that it has an impact on glucose homeostasis. After 15 days of treatment with MPM, animals on a high-fructose diet have a faster plasma glucose clearance (Fig 7A) and a better sensitivity to insulin (Fig 7B). This effect is reversible. Indeed, the sensitivity to insulin of animals which developed insulin resistance after one month of a high-fructose diet become normal again after one month of treatment with MPM. In contrast, stopping the administration of MPM to animals on a high-fructose diet decreases their sensitivity to insulin leading to insulin resistance (Fig 7C).

Metaflammation has an important impact in the pathogenesis of insulin resistance and type 2 diabetes mellitus. There is a strong correlation between the levels of pro-inflammatory cytokines like IL-6 and TNF*α* and perturbation in glucose management (Tilg et al., 2006, Nature Rev 6: 772-783). The production of cytokines TNF*α*, IL-1*β*, IL-6 and GM-CSF significantly decreases following MPM consumption (Fig. 2A). These results indicate that inhibition of the Th₁₇ pathway by MPM leads to better glucose homeostasis and insulin sensitivity.

### EXAMPLE 9

### Effect of MPM on systolic blood pressure (SBP) of spontaneously hypertensive rats (SHR).

Spontaneously hypertensive rats (SHR, 6 weeks old) were maintained in a 12-hour light/dark cycle and consumed standard diet and water *ad libitum.* 10 rats were randomly assigned to various treatment groups. Rats were maintained on a regular diet for 2 weeks in order to allow the development of hypertension. Hypertensive rats were then treated for 3 weeks by daily intragastric gavages with water (placebo group, 1 ml/day), MPM (200mg/day) or enalapril-malate (10 mg/kg; a known hypotensive agent). Daily intragastric gavages were then stopped for the last week of the experiment. Systolic blood pressure was measured weekly with the automated RTBP2000 Tail Blood Pressure system (Kent Scientific, Torrington, CT, USA). An average of 3 measurements was taken as initial mean SBP. Data was acquired and analyzed with Biopac Student Lab Pro® software version 3.6.1 (Biopac System, USA).

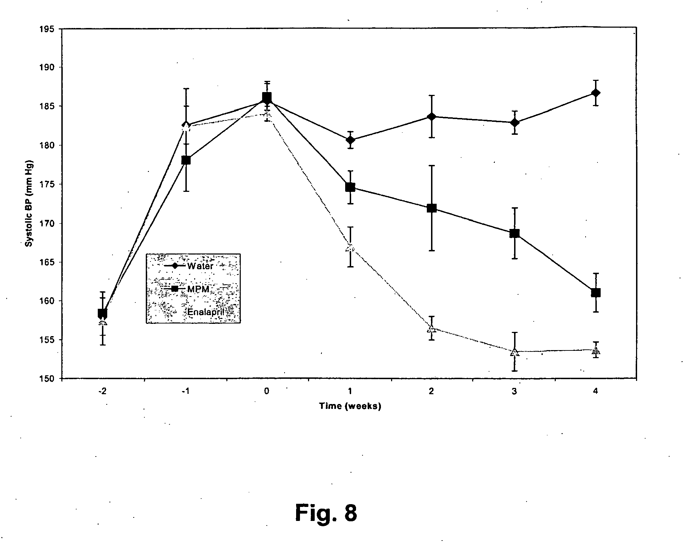

In this model, an initial period of 2 weeks was necessary to achieve high systolic blood pressure and after which treatments were started. Enalapril- treated group had a normalized SBP going from 184 mm Hg to around 153 mm Hg after 4 weeks (17% reduction) while a decrease of SBP was also observed for the MPM group with a maximum reduction of 14 % at week 4 (Fig. 8) comparable to Enalapril. These results clearly show the potential of MPM for SBP reduction.

Hypertension acts as a major determinant of endothelial dysfunction and vascular damage. It promotes inflammatory activation of endothelial cells, recruitment of inflammatory cells in the arterial wall and activation of vascular resident elements. In agreement with this theory, it has been shown that an inflammatory response can develop in the arteries of animal models of hypertension characterized by the expression of IL-6, IL-1, TNFα, MCP-1 and adhesion molecules (ICAM-1, VCAM-1).

Recent studies in this animal model of hypertension showed that the recruitment of immune cells in the kidney can be prevented. This phenomenon is accompanied by a complete abrogation of hypertension development in spontaneously hypertensive rats (Pauletto et al. 2006, Nephro Dial Transplant 21: 850-853). Even if the factors inducing the inflammatory response in the kidney are not defined, it could be assumed that the infiltration of immune cells and the oxidative stress in the renal interstitium play a pathogenic role in the future development of hypertension.

The production of cytokines TNF*α*, IL-1*β*, IL-6 and GM-CSF significantly decreases following MPM consumption (Fig. 2A). These results shows that MPM prevent hypertension through the Th₁₇ pathway by negatively regulating the granulocyte-dependent response and thus preventing recruitment of immune cells in the kidney.

### EXAMPLE 10

### Effect of MPM on the expression of different genes human keratinocytes (HEKA).

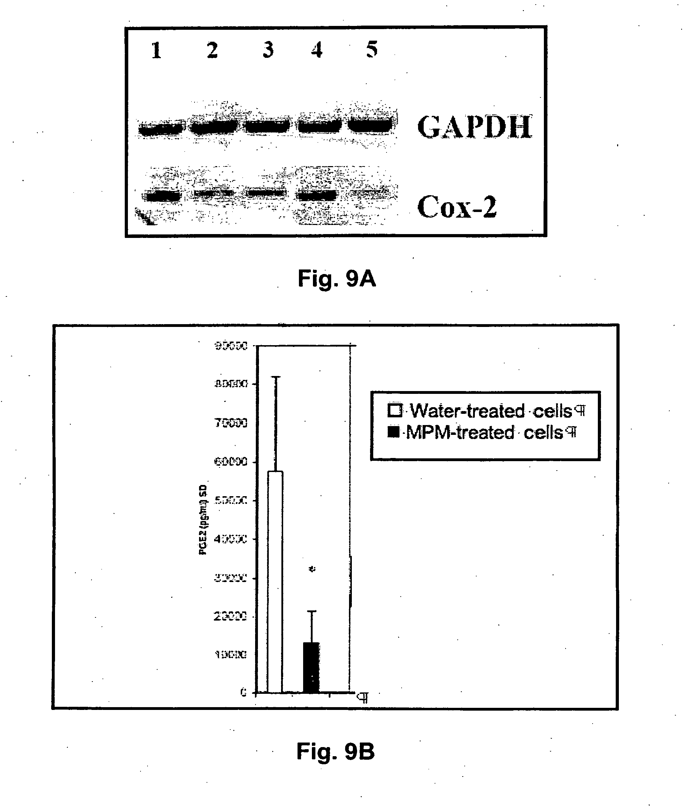

Sub-confluent human keratinocytes (HEKA - Cascade Biologics) were exposed for 16-hour with the following : lane 1, water (control); lane 2, lipopolysaccharide (LPS, 10 ug/ml); lane 3, exopolysaccharide (10ug/ml); lane 4, calcium (1.5 mM); lane 5, MPM diluted in PBS 1:1000 (Fig 9A). Cells were then harvest and RNA was isolated using the RNeasy™ Qiagen kit. Semiquantitative PCR was performed with specific oligonucleotides to detect GAPDH (control) and COX-2. Non-differentiated and differentiated keratinocytes (EPI-200 model™ - Mattek) were exposed for 24-hour with water (control) or MPM diluted in PBS 1:100 (Fig 9B). PGE2 production in the supernatant was measured by ELISA (Cayman Cat 514010).

Topical activity of MPM was monitored by means of sensitive and meaningful biomarkers of skin integrity such as prostaglandin E2 (PGE₂) and cyclooxygenase 2 (COX-2), both guardians of the degree of epithelial homeostasis. The expression of COX-2 was reduced following an MPM exposure as shown in Figure 9A. To push further this observation, the consequence of COX-2 reduction is a lower basal level of PGE₂ biosynthesis by about 75% after a 24-hour exposure in human keratinocytes exposed to MPM (Fig 9B). The decrease in PGE₂ production following MPM exposure is responsible of the inhibition of the expansion and survival of Th₁₇ cells since high level of PGE₂ can exacerbate the inflammatory process through the IL23/IL-17 axis maintaining a pro-inflammatory circuit. This indicate that COY-2 inhibition play an important role in MPM anti-inflammatory effect.

### EXAMPLE 11

### Effect of MPM in preventive and therapeutic administration in an Atopic Contact Dermatitis (ACD) model.

Water (100µl; control) or MPM (1 mg/g) were administered p.o. each day in the morning for 1 week in prevention. After this period, the murine model of ACD was done as follows: abdomen hairs of CD-1 mice were removed and the sensitization phase was done with the application of 100 *µ*L of oxazolone 5 % in acetone on the hairless abdomen. After 4 days, the elicitation phase (first challenge) was initiated with application of 50 *µ*l of oxazolone 5% in acetone on the right ear (25 *µ*l each side of the ear). The second challenge was done 7 days after the first challenge with the same procedure. The water or MPM was administered *per os* each day during the entire experiment. The ear thickness was measured each day.

The murine model of ACD was done as follows: abdomen hairs of CD-1 mice were removed and the sensitization phase was done with the application of 100 *µ*L of oxazolone 5 % in acetone on the hairless abdomen. After 4 days, the elicitation phase (first challenge) was initiated with application of 50 *µ*l of oxazolone 5% in acetone on the right ear (25 *µ*l each side of the ear). The second challenge was done 7 days after the first challenge with the same procedure. In the therapeutic model, water (100µl; control) or MPM (1 mg/g) were administered p.o. each day in the morning during the entire experiment only after the first challenge. The ear thickness was measured each day.

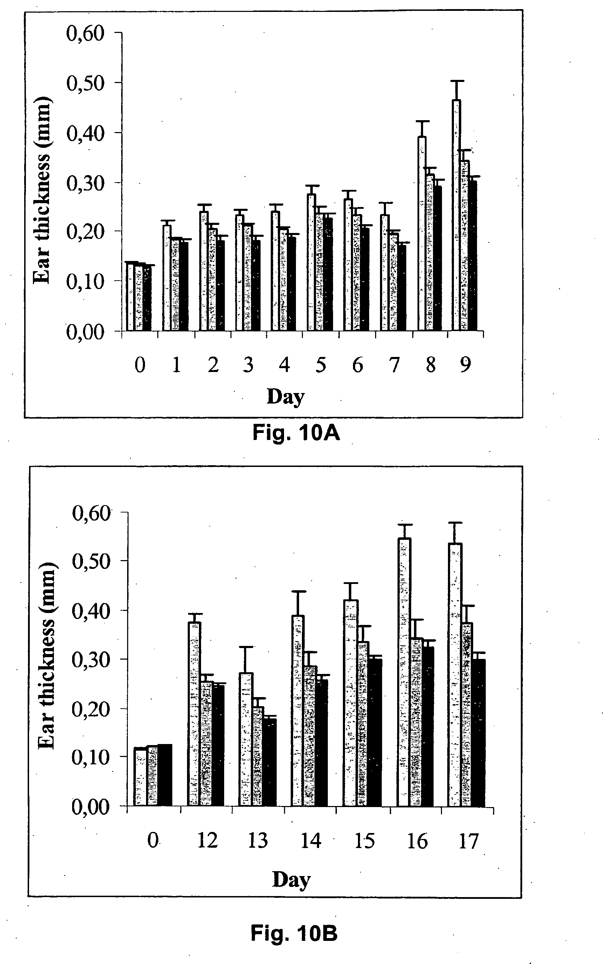

MPM and hydrocortisone administered p.o. either in a preventive (Figure 10A) or a therapeutic fashion (Figure 10B) reduced the inflammation with similar efficiency as demonstrated by the reduction of ear redness and thickness. The effect of MPM in therapeutic model showed that a certain period of time is required to overcome existing inflammation. This indicates that MPM possesses an anti-inflammatory effect in an existing disease and is not only a preventive treatment.

MPM significantly reduces the inflammation in this model and this anti-inflammatory effect is comparable to those obtained with an oral hydrocortisone treatment without the immunosuppression observed following hydrocortisone administration (Beaulieu et al., 2007, J Inflamm 4: 6). The reduction of inflammation in this model is observed in both preventive and therapeutic way. The preventive way had better impact, more efficient and earlier than the therapeutic way.

### EXAMPLE 12

### Effect of MPM on stimulation of the body natural defenses in healthy animals

Male C57BI/6J mice were treated daily by intragastric gavage (7 days a week) with water (control group, 100µl) or MPM (1mg/g). After 28 days of treatment, a spontaneous foreskin Staphylococcus *aureus* infection occurred in the animal house. Animals infected were counted and treated.

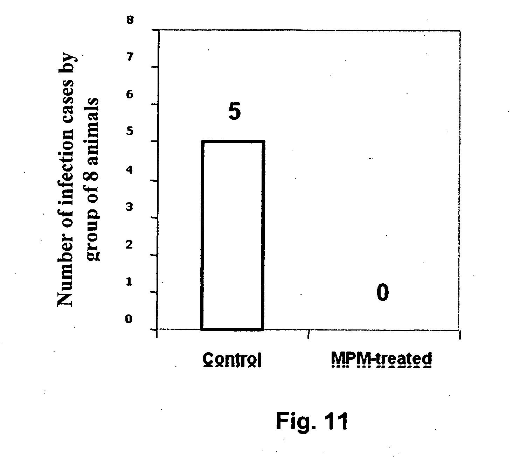

MPM-treated animals (0/8, Fig.11) didn't get infected by Staphylococcus *aureus* compare to untreated animals (5/8, Fig. 11). Therefore, MPM significantly protect against spontaneous infections by stimulating the body natural defenses.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

## Claims

1. A malleable protein matrix (MPM) for use in the treatment of a condition selected from the group consisting of, arthritis, obesity, insulin resistance, type 2 diabetes, autoimmune disease, hypertension, and hyperlipidemia in a subject, wherein:
• said MPM is obtained by triggering agglomeration of whey proteins from fermented whey, said whey proteins being then retrieved; and
• said MPM modulates the biological activity of IL-17-producing cells.

2. A malleable protein matrix (MPM) for use according to claim 1, wherein said subject is a human.

3. A malleable protein matrix (MPM) for use according to claim 1, wherein said subject is an animal.

4. A malleable protein matrix (MPM) for use according to any one of claims 1-3, wherein said infection is selected from the group consisting of a bacterial infection, a viral infection and fungal infection.

5. A malleable protein matrix (MPM) for use according to any one of claims 1-3, wherein said MPM reduces expression of a cytokine selected from the group consisting of IL-12, IL-1β, IL-6, transforming growth factor (TNFα), granulocyte-macrophage colony-stimulating factor (GM-CSF) and interferon gamma (IFNγ).

6. A malleable protein matrix (MPM) for use according to any one of claims 1-3, wherein said MPM decreases levels of plasma triglycerides or cholesterol.

7. A malleable protein matrix (MPM) for use according to any one of claims 1-3, wherein said MPM helps control weight or body composition.

8. A malleable protein matrix (MPM) for use according to any one of claims 1-3, wherein said MPM decreases the expression of cyclooxygenase 2 (COX-2) or production of prostaglandin E2 (PGE₂).

9. A malleable protein matrix (MPM) for use according to any one of claims 1-3, wherein said MPM increases polymorphonuclear cells or CD4⁺ cells population.

10. A malleable protein matrix (MPM) for use according to any one of the preceding claims, wherein in the treatment an additional immunosuppressive or anti-inflammatory agent is to be administered, concurrently or prior to / subsequent to the administration of MPM, within the same or different dosage.

## Patentansprüche

1. Plastische Proteinmatrix (MPM) zur Verwendung in der Behandlung einer Erkrankung, ausgewählt aus der Gruppe Arthritis, Fettsucht, Insulinresistenz, Diabetes Typ 2, Autoimmunkrankheiten, Hypertension und Hyperlipidemie in einem Patienten, worin
• die MPM erhalten wird durch Auslösen von Agglomeration von Molkenproteinen aus vergärter Molke, wobei die Molkenproteine dann eingesammelt werden; und
• die MPM die biologische Aktivität von IL-17-produzierenden Zellen moduliert.

2. Plastische Proteinmatrix (MPM) zur Verwendung gemäß Anspruch 1, wobei der Patient ein Mensch ist.

3. Plastische Proteinmatrix (MPM) zur Verwendung gemäß Anspruch 1, wobei der Patient ein Tier ist.

4. Plastische Proteinmatrix (MPM) zur Verwendung gemäß irgendeinem der Ansprüche 1 bis 3, wobei die Infektion ausgewählt ist aus der Gruppe bakterielle Infektion, virale Infektion und Pilzinfektion.

5. Plastische Proteinmatrix (MPM) zur Verwendung gemäß irgendeinem der Ansprüche 1 bis 3, wobei das MPM die Expression eines Cytokins verringert, ausgewählt aus der Gruppe IL-12, IL-1 β, IL-6, Transforming Growth Factor (TNFα), Granulocyte macrophage colony-stimulating factor (GM-CSF) und Interferon Gamma (IFNγ).

6. Plastische Proteinmatrix (MPM) zur Verwendung gemäß irgendeinem der Ansprüche 1 bis 3, wobei das MPM die Spiegel von Plasmatriglyceriden oder Cholesterin verringert.

7. Plastische Proteinmatrix (MPM) zur Verwendung gemäß irgendeinem der Ansprüche 1 bis 3, wobei das MPM hilft, Gewicht oder Körperzusammensetzung zu steuern.

8. Plastische Proteinmatrix (MPM) zur Verwendung gemäß irgendeinem der Ansprüche 1 bis 3, wobei das MPM die Expression von Cyclooxygenase 2 (COX-2) oder die Produktion von Prostaglandin E2 (PGE₂) verringert.

9. Plastische Proteinmatrix (MPM) zur Verwendung gemäß irgendeinem der Ansprüche 1 bis 3, wobei das MPM die Population von polymorphonuklearen Zellen oder CD4⁺-Zellen erhöht.

10. Plastische Proteinmatrix (MPM) zur Verwendung gemäß irgendeinem der vorhergehenden Ansprüche, wobei in der Behandlung ein weiteres immunosuppressives oder entzündungshemmendes Mittel verabreicht werden soll, gleichzeitig mit oder vor/nach der Verabreichung von MPM, mit der gleichen oder einer anderen Dosierung.

## Revendications

1. Une matrice protéique malléable (MPM) destinée à une utilisation dans le traitement d'une pathologie sélectionnée dans le groupe se composant d'arthrite, obésité, résistance à l'insuline, diabètes de type 2, maladie autoimmune, hypertension et hyperlipidémie chez un sujet où :
• ladite MPM est obtenue par le déclenchement d'une agglomération de protéines de lactosérum provenant de lactosérum fermenté, lesdites protéines de lactosérum étant ensuite récupérées, et·
• ladite MPM module l'activité biologique de cellules productrices de IL-17.

2. Une matrice protéique malléable (MPM) destinée à une utilisation selon la revendication 1, où ledit sujet est un humain.

3. Une matrice protéique malléable (MPM) destinée à une utilisation selon la revendication 1, où ledit sujet est un animal.

4. Une matrice protéique malléable (MPM) destinée à une utilisation selon l'une quelconque des revendications 1 à 3, où ladite infection est sélectionnée dans le groupe se composant d'une infection bactérienne, d'une infection virale et d'une infection fungique.

5. Une matrice protéique malléable (MPM) destinée à une utilisation selon l'une quelconque des revendications 1 à 3, où ladite MPM réduit l'expression d'une cytokine sélectionnée dans le groupe se composant de IL-12, IL-1β, IL-6, facteur de croissance transformant (TNFα),facteur de stimulation de colonies de granulocytes-macrophages (GM-CSF) et interféron gamma (IFNγ).

6. Une matrice protéique malléable (MPM) destinée à une utilisation selon l'une quelconque des revendications 1 à 3, où ladite MPM diminue des niveaux de triglycérides du plasma ou de cholestérol.

7. Une matrice protéique malléable (MPM) destinée à une utilisation selon l'une quelconque des revendications 1 à 3, où ladite MPM aide à contrôler le poids ou la composition d'un corps.

8. Une matrice protéique malléable (MPM) destinée à une utilisation selon l'une quelconque des revendications 1 à 3, où ladite MPM diminue l'expression de la cyclo-oxygénase 2 (COX- 2) ou la production de prostaglandine E2 (PGE₂).

9. Une matrice protéique malléable (MPM) destinée à une utilisation selon l'une quelconque des revendications 1 à 3, où ladite MPM augmente la population de cellules polymorphonucléaires ou de cellules CD4⁺.

10. Une matrice protéique malléable (MPM) destinée à une utilisation selon l'une quelconque des Revendications précédentes, où, dans le traitement, un agent immunosuppresseur ou anti-inflammatoire additionnel doit être administré simultanément ou avant / après l'administration de MPM dans le même dosage ou un dosage différent.
